Europäisches Patentamt

European Patent Office

Office européen des brevets

(19)

(11) Veröffentlichungsnummer: **0 339 483**
**A2**

(12) **EUROPÄISCHE PATENTANMELDUNG**

(21) Anmeldenummer: 89107097.1

(22) Anmeldetag: 20.04.89

(51) Int. Cl.⁴: **C07K 5/02 , C07K 5/06 ,**
**C07D 233/64 , C07D 211/58 ,**
**A61K 37/64 , A61K 31/395 ,**
**C07C 143/74 , A61K 31/18**

Die Bezeichnung der Erfindung wurde geändert (Richtlinien für die Prüfung im EPA, A-III, 7.3).

(30) Priorität: 28.04.88 DE 3814325

(43) Veröffentlichungstag der Anmeldung:
02.11.89 Patentblatt 89/44

(84) Benannte Vertragsstaaten:
AT BE CH DE ES FR GB IT LI NL SE

(71) Anmelder: MERCK PATENT GESELLSCHAFT
MIT BESCHRÄNKTER HAFTUNG
Frankfurter Strasse 250 Postfach 4119
D-6100 Darmstadt(DE)

(72) Erfinder: Raddatz, Peter, Dr.
Grafenstrasse 27
D-6100 Darmstadt(DE)
Erfinder: Schmitges, Claus J., Dr.
Karolinger Strasse 5
D-6114 Gross-Umstadt(DE)
Erfinder: Minck, Klaus-Otto, Dr.
Büchestrasse 8
D-6105 Ober-Ramstadt(DE)

(54) **Renin inhibierende Aminosäure-Derivate.**

(57) Neue Aminosäurederivate der Formel I
R¹-Z-NR²-CHR³-CR⁴-CH₂-CR⁵-R⁶-Y    I
worin R¹ bis R⁶, Z, und Y die in Patentanspruch 1 angegebenen Bedeutungen haben,
sowie ihre Salze hemmen die Aktivität des menschlichen Plasmarenins.

EP 0 339 483 A2

## Aminosäurederivate

Die Erfindung betrifft neue Aminosäurederivate der Formel I

$R^1$-Z-$NR^2$-$CHR^3$-$CR^4$-$CH_2$-$CR^5R^6$-Y     I

worin

$R^1$ H, $R^7$-O-$C_mH_{2m}$-CO-, $R^7$-$C_mH_{2m}$-O-CO-, $R^7$-$C_mH_{2m}$-CO-, $R^7$-$SO_2$-, $R^8R^9$N-$C_mH_{2m}$-CO-, $R^{10}$-NH-C(=NH)-NH-$C_mH_{2m}$-CO-, $R^8$OOC-$C_mH_{2m}$-CO-, $R^8O_3$S-$C_mH_{2m}$-CO- oder $R^{11}$-$C_mH_{2m}$-(T)$_s$-(V)$_y$-$C_nH_{2n}$-L($R^7$-$C_pH_{2p}$)-$C_rH_{2r}$-CO-,

Z 1 bis 4 peptidartig miteinander verbundene Aminosäurereste ausgewählt aus der Gruppe bestehend aus Abu, Ada, Ala, βAla, Arg, Asn, Asp, Bia, Cal, Dab, Gln, Glu, Gly, His, N(im)-A-His, Hph, Ile, Leu, tert.-Leu, Lys, Mal, Met, αNal, βNal, Nbg, Nle, Orn, Phe, Pia, Pro, Pyr, Ser, Thr, Tia, Tic, Trp, Tyr und Val,

Y -CN, -$CH_2$-$NR^{12}R^{13}$, -$CH_2$-$NR^{12}$-$SO_2R^{14}$, -$CH_2$-$NR^{12}$-$COR^{14}$, -$CH_2$-$NR^{12}$-CO-NH-$R^{14}$, oder -$CH_2$-$NR^{12}$-CS-NH-$R^{14}$,

$R^2$, $R^5$, $R^6$, $R^8$, $R^9$ und $R^{13}$ jeweils H oder A,

$R^3$, $R^7$, $R^{11}$, $R^{12}$ und $R^{14}$ jeweils H, A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

$R^{11}$ auch $R^8$O-, $R^8R^9$N-, $R^8$OOC- oder $A_3N^\oplus$ An$^\ominus$,

$R^4$ (H, OH), (H, $NH_2$) oder =O,

$R^{13}$ H, A oder CN,

L CH oder N,

T O, NH oder NA,

V CHOH, CO, S, SO oder $SO_2$,

$R^8R^9$N und $NR^{12}R^{13}$ auch jeweils eine unsubstituierte oder eine durch A, OH, $NH_2$, NHA, $NA_2$, NHAc, NH-CO-$C_xH_{2x}$-O-$R^{15}$, NH-CO-O-$C_xH_{2x}$-$R^{15}$, Hydroxyalkyl, COOH, COOA, $CONH_2$, Aminoalkyl, HAN-alkyl, $A_2$N-alkyl, $A_3N^\oplus$alkyl An$^\ominus$, NH-CO-$NH_2$, NH-CO-NHA, Guanidinyl oder Guanidinyl-alkyl substituierte Pyrrolidino-, Piperidino-, Morpholino- oder Piperazino-gruppe,

$R^{15}$ A oder Ar-alkyl,

s und y jeweils 0 oder 1,

m, n, p, r und x jeweils 0, 1, 2, 3, 4, 5, 6, 7, 8, 9 oder 10,

Ar unsubstituiertes oder ein- oder mehrfach durch A, OA, Hal, $CF_3$, OH, $NO_2$, Hydroxyalkyl, $NH_2$, NHA, $NA_2$, NHAc, SA, SO-A, $SO_2$-A, $SO_2NH_2$, $SO_2$NHA, COOH, COOA, $CONH_2$, CN, Aminoalkyl, HAN-alkyl, $A_2$N-alkyl, $A_3N^\oplus$alkyl An$^\ominus$ und/oder Guanidinyl-alkyl substituiertes Phenyl oder unsubstituiertes Naphthyl,

Het einen gesättigten oder ungesättigten 5- oder 6-gliedrigen heterocyclischen Rest mit 1-4 N-, O- und/oder S-Atomen, der mit einem Benzolring kondensiert und/oder ein- oder mehrfach durch A, OA, Hal, $CF_3$, OH, $NO_2$, Carbonylsauerstoff, $NH_2$, NHA, $NA_2$, NHAc, SA, SO-A, $SO_2$-A, $SO_2NH_2$, $SO_2$NHA, COOH, COOA, $CONH_2$, CN, NH-$SO_2$-A, Ar, Ar-alkyl, Ar-alkenyl, Hydroxyalkyl, Aminoalkyl, HAN-alkyl und/oder $A_2$N-alkyl substituiert sein kann und/oder dessen N- und/oder S-Heteroatome auch oxydiert sein können,

Hal F, Cl, Br oder J,

Ac A-CO-, Ar-CO-, Ar-alkyl-CO- oder A-NH-CO-,

An$^\ominus$ ein Anion, das auch fehlen kann, wenn stattdessen eine in der Verbindung der Formel I enthaltene Carboxygruppe in Form eines Carboxylatanions vorliegt,

-alkyl- eine Alkylengruppe mit 1-8 C-Atomen und

A Alkyl mit 1-8 C-Atomen bedeuten,

worin ferner an Stelle einer oder mehrerer -NH-CO-Gruppen auch eine oder mehrere -NA-CO-Gruppen stehen können,

sowie deren Salze.

Ähnliche Verbindungen sind aus der EP-A-249096 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z.B. nach der Methode von F. Fyhrquist et al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z.B. Pepsin und Kathepsin D) sind in der Regel etwa 100 bis 1000mal so hohe Konzentrationen dieser Verbindungen notwendig wie für die Renin-Hemmung. Die Wirkung der Verbindun-

2

gen auf den Blutdruck und/oder auf die Herzfrequenz sowie die Hemmung der Reninaktivität im Blutplasma können ferner an wachen Affen, z.B. weiblichen Affen (Macaca fascicularis) ermittelt werden; dabei können Blutdruck und Herzfrequenz in Anlehnung an die Methode von M.J. Wood et al., J. Hypertension 4, 251-254 (1985) gemessen werden. Zur Stimulierung der Reninaktivität werden die Tiere dabei zweckmäßig mit einem Saluretikum vorbehandelt. Blutproben zur Bestimmung der Plasma-Reninaktivität können durch Punktion der Vena femoralis gewonnen werden.

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen. Solche diagnostische Tests können ausgeführt werden ähnlich wie es in der EP-A-77 028 angegeben ist.

Die vor- und nachstehend aufgeführten Abkürzungen von Aminosäureresten stehen für die Reste $-NR'-R''-CO-$, in der Regel $-NH-CHR-CO-$ (worin R, $R'$ und $R''$ die für jede Aminosäure bekannte spezifische Bedeutung haben) folgender Aminosäuren:

| | |
|---|---|
| Abu | 2-Aminobuttersäure |
| Ada | 3-(1-Adamantyl)-alanin |
| Ala | Alanin |
| ßAla | ß-Alanin |
| Arg | Arginin |
| Asn | Asparagin |
| Asp | Asparaginsäure |
| Bia | 3-(2-Benzimidazolyl)-alanin |
| Cal | 3-Cyclohexylalanin |
| Dab | 2,4-Diaminobuttersäure |
| Gln | Glutamin |
| Glu | Glutaminsäure |
| Gly | Glycin |
| His | Histidin |
| N(im)-A-His | in 1- oder 3-Stellung des Imidazolrings durch A substituiertes Histidin |

| | |
|---|---|
| Hph | Homophenylalanin (2-Amino-4-phenyl-butter-säure) |
| Ile | Isoleucin |
| Leu | Leucin |
| tert.-Leu | tert.-Leucin |
| Lys | Lysin |
| Mal | 3-(p-Methoxyphenyl)-alanin |
| Met | Methionin |
| αNal | 3-(α-Naphthyl)-alanin |
| ßNal | 3-(ß-Naphthyl)-alanin |
| Nbg | 2-Norbornyl-glycin |
| Nle | Norleucin |
| N-Me-His | N-Methyl-histidin |
| N-Me-Phe | N-Methyl-phenylalanin |
| Orn | Ornithin |
| Phe | Phenylalanin |
| Pia | 3-(Piperidyl)-alanin [z.B. 2-Pia = 3-(2-Piperidyl)-alanin] |
| Pro | Prolin |
| Pya | 3-(Pyridyl)-alanin [z.B. 3-Pya = 3-(3-Pyridyl)-alanin] |
| Ser | Serin |
| Thr | Threonin |
| Tia | 3-(Thienyl)-alanin [z.B. 2-Tia = 3-(2-Thienyl)-alanin] |
| Tic | 1,2,3,4-Tetrahydroisochinolin-1-carbonsäure |
| Trp | Tryptophan |
| Tyr | Tyrosin |
| Val | Valin. |

Ferner bedeutet nachstehend:

4

| BOC | tert.-Butoxycarbonyl |
|---|---|
| BCM | Benzyloxymethyl |
| imi-BOM | Benzyloxymethyl in 1-Stellung des Imidazolrings |
| CBZ | Benzyloxycarbonyl |
| DCCI | Dicyclohexylcarbodiimid |
| DMF | Dimethylformamid |
| DNP | 2,4-Dinitrophenyl |
| imi-DNP | 2,4-Dinitrophenyl in 1-Stellung des Imidazolrings |
| ETOC | Ethoxycarbonyl |
| FMOC | 9-Fluorenylmethoxycarbonyl |
| HOBt | 1-Hydroxybenzotriazol |
| IPOC | Isopropoxycarbonyl |
| POA | Phenoxyacetyl |
| THF | Tetrahydrofuran. |

Sofern die vorstehend genannten Aminosäuren in mehreren enantiomeren Formen auftreten können, so sind vor- und nachstehend, z.B. als Bestandteil der Verbindungen der Formel I, alle diese Formen und auch ihre Gemische (z.B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Gegenstand der Erfindung ist ferner ein Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Carbonsäure der Formel II

$R^1$-$G^1$-OH    II

worin $G^1$

    (a) fehlt,

    (b) Z,

    (c) $Z^1$ bedeutet

oder eines ihrer reaktionsfähigen Derivate

mit einer Aminoverbindung der Formel III

H-$G^2$-$NR^2$-$CHR^3$-$CR^4$-$CH_2$-$CR^5R^6$-Y    III

worin $G^2$

    (a) Z bedeutet,

    (b) fehlt,

    (c) $Z^2$ bedeutet und

$Z^1$ + $Z^2$ zusammen Z bedeuten,

umsetzt,

und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine freie Aminogruppe durch Behandeln mit einem acylierenden Mittel acyliert und/oder zur Herstellung einer Verbindung der Formel I, $R^4$ = (H, OH) oder (H, $NH_2$), ein Aminoketosäure- derivat der Formel I, $R^4$ = O, reduziert oder reduktiv aminiert und/oder einen Rest $R^1$ in einen anderen Rest $R^1$ umwandelt und/oder eine Verbindung der Formel I durch Behandeln mit einer Säure in eines ihrer Salze überführt.

Vor- und nachstehend haben die Reste bzw. Parameter $R^1$ bis $R^{15}$, Z, Y, L, T, V, m, n, p, r, s, x, y, Ar, Het. Hal, Ac, An, A, $G^1$, $G^2$, $Z^1$, und $Z^2$ die bei den Formeln I, II oder III angegebenen Bedeutungen, falls nicht ausdrücklich etwas anderes angegeben ist.

In den vorstehenden Formeln hat A 1-8, vorzugsweise 1, 2, 3 oder 4 C-Atome. A bedeutet vorzugsweise Methyl, weiterhin Ethyl, Propyl, Isopropyl, Butyl, Isobutyl, sek.-Butyl, oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2- methylpropyl, 1,1,2- oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl, aber auch z.B. 1-, 2- oder 3-Methylcyclopentyl, 1-, 2-, 3- oder 4-Methylcyclohexyl.

Dementsprechend bedeutet Cycloalkylalkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohex-

5

ylethyl, aber auch z.B. 1-, 2- oder 3-Methylcyclopentylmethyl, 1-, 2-, 3- oder 4-Methylcyclohexylmethyl.

Bicycloalkyl bedeutet vorzugsweise 1- oder 2-Dekalyl, 2-Bicyclo[2,2,1]heptyl oder 6,6-Dimethyl-2-bicyclo[3,1,1]heptyl.

Tricycloalkyl bedeutet vorzugsweise 1-Adamantyl.

Hal bedeutet vorzugsweise F, Cl oder Br, aber auch J.

Ac bedeutet vorzugsweise A-CO-, wie Acetyl, Propionyl oder Butyryl, Ar-CO- wie Benzoyl, o-, m- oder p-Methoxybenzoyl oder 3,4-Dimethoxybenzoyl, A-NH-CO- wie N-Methyl-oder N-Ethylcarbamoyl.

Ar bedeutet vorzugsweise Phenyl, ferner bevorzugt o-, m- oder p-Tolyl, o-, m- oder p-Ethylphenyl, o-, m- oder p-Methoxyphenyl, o-, m- oder p-Fluorphenyl, o-, m- oder p-Chlorphenyl, o- m- oder p-Bromphenyl, o-, m- oder p-Jodphenyl, o-, m- oder p-Trifluormethylphenyl, o-, m-oder p-Hydroxyphenyl, o-, m- oder p-Sulfamoylphenyl, 2,3-, 2,4-, 2,5-, 2,6-, 3,4- oder 3,5-Dimethoxyphenyl, 3,4,5-Trimethoxyphenyl, o-, m- oder p-Aminophenyl, o-, m- oder p-Aminomethylphenyl, o-, m- oder p-Dimethylaminomethylphenyl, o-, m- oder p-Guanidinomethylphenyl, 1- oder 2-Naphthyl.

Dementsprechend bedeutet Ar-alkyl vorzugsweise Benzyl, 1- oder 2-Phenylethyl, o-, m- oder p-Methylbenzyl, 1-oder 2-o-, -m- oder -p-Tolylethyl, o-, m- oder p-Ethylbenzyl, 1- oder 2-o-, -m- oder -p-Ethylphenylethyl, o-, m- oder p-Methoxybenzyl, 1- oder 2-o-, -m- oder -p-Methoxyphenylethyl, o-, m- oder p-Fluorbenzyl, 1- oder 2-o-, -m- oder -p-Fluorphenylethyl, o-, m- oder p-Chlorbenzyl, 1- oder 2-o-, -m- oder -p-Chlorphenylethyl, o-, m- oder p-Brombenzyl, 1- oder 2-o-, -m- oder -p- Bromphenylethyl, o-, m- oder p-Jodbenzyl, 1- oder 2-o-, -m-oder -p-Jodphenylethyl, o-, m- oder p-Trifluormethylbenzyl, o-, m- oder p-Hydroxybenzyl, 2,3-, 2,4-, 2,5-, 2,6- 3,4- oder 3,5-Dimethoxybenzyl, 3,4,5-Trimethoxybenzyl, o-, m- oder p-Aminobenzyl, o-, m- oder p-Aminomethylbenzyl, o- m- oder p-Dimethylaminomethylbenzyl, o-, m- oder p-Guanidinomethylbenzyl, 1- oder 2-Naphthylmethyl.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4-oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 2,1,5-Thiadiazol-3- oder -4-yl, 2-, 3- 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6-oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolyl, 2-, 4-, 5-, 6- 7- oder 8-Chinazolyl. Die heterocyclischen Reste können auch teilweise oder vollständig hydriert sein. Het kann also z.B. auch bedeuten 2,3-Dihydro-2-, -3-, -4- oder -5-furyl, 2,5-Dihydro-2-, -3-, -4- oder -5-furyl, Tetrahydro-2- oder -3-furyl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1,2,3,6-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4- pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro- 1-, -2-, -3-, -4-, -5-, -6-, -7- oder -8-chinolyl, 1,2,3,4-Tetrahydro-1-, -2-, -3-, -4-, -5-, -6-, -7- oder 8-isochinolyl.

Die heterocyclischen Reste können auch wie angegeben substituiert sein. Het kann z.B. bevorzugt auch bedeuten: 2-Amino-4-thiazolyl, 4-Carboxy-2-thiazolyl, 4-Carbamoyl-2-thiazolyl, 4-(2-Aminoethyl)-2-thiazolyl, 4-Amino-2-methyl-5-pyrimidinyl, 2-Amino-5,6-dimethyl-3-pyrazinyl, 4-Carbamoylpiperidino, ferner z.B. 3-, 4- oder 5-Methyl-2-furyl, 2-, 4- oder 5-Methyl-3-furyl, 2,4-Dimethyl-3-furyl, 5-Nitro-2-furyl, 5-Styryl-2-furyl, 3-, 4- oder 5-Methyl-2-thienyl, 2-, 4- oder 5-Methyl-3-thienyl, 3-Methyl-5-tert.-butyl-2-thienyl, 5-Chlor-2-thienyl, 5-Phenyl-2- oder -3-thienyl, 1-, 3-, 4- oder 5-Methyl-2-pyrrolyl, 1-Methyl-4- oder -5-nitro-2-pyrrolyl, 3,5-Dimethyl-4-ethyl-2-pyrrolyl, 4-Methyl-5-pyrazolyl, 5-Methyl-3-isoxazolyl, 3,4-Dimethyl-5-isoxazolyl, 4- oder 5-Methyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 3-, 4-, 5- oder 6-Chlor-2-pyridyl, 2-, 4-, 5- oder 6-Chlor-3-pyridyl, 2- oder 3-Chlor-4-pyridyl, 2,6-Dichlorpyridyl, 2-Hydroxy-3-, -4-, -5- oder -6-pyridyl (= 1H-2-Pyridon-3-, -4-, -5- oder -6-yl), 5-Phenyl-1H-2-pyridon-3-yl, 5-p-Methoxyphenyl-1H-2-pyridon-3-yl, 2-Methyl-3-hydroxy-4-hydroxymethyl-5-pyridyl, 2-Hydroxy-4-amino-6-methyl-3-pyridyl, 3-N'-Methylureido-1H-4-pyridon-5-yl, 4-Methyl-2-pyrimidinyl, 4,6-Dimethyl-2-pyrimidinyl, 2-, 5- oder 6-Methyl-4-pyrimidinyl, 2,6-Dimethyl-4-pyrimidinyl, 2,6-Dihydroxy-4-pyrimidinyl, 5-Chlor-2-

6

methyl-4-pyrimidinyl, 3-Methyl-2-benzofuryl, 2-Ethyl-3-benzofuryl, 7-Methyl-2-benzothienyl, 1-, 2-, 4-, 5-, 6- oder 7-Methyl-3-indolyl, 1-Methyl-5- oder -6-benzimidazolyl, 1-Ethyl-5- oder -6- benzimidazolyl, 3-, 4-, 5-, 6- , 7- oder 8-Hydroxy-2-chinolyl, 2-Oxo-pyrrolidino, 2-Oxo-piperidino, 2,5-Dioxopyrrolidino, 3-Benzyl-2,5-dioxo-pyrrolidino.

$R^1$ ist allgemein vorzugsweise $R^8 R^9 N-C_m H_{2m}-CO-$ oder $R^{11}-C_m H_{2m}-(T)_s-(V)_y-C_n H_{2n}-L(R^7-C_p H_{2p})-C_r H_{2r}-CO-$, ferner bevorzugt $R^7-C_m H_{2m}-O-CO-$, $R^7-C_m H_{2m}-CO-$ oder $R^8 OOC-C_m H_{2m}-CO-$.

Die Gruppe Z besteht vorzugsweise aus einem oder zwei der angegebenen Aminosäurereste; sie kann jedoch auch drei oder vier Aminosäurereste enthalten. Vorzugsweise bedeutet Z His oder Phe-His, ferner bevorzugt Cal-His, Mal-His, αNal-His, ßNal-His, Phe-Abu, Phe-Ala, Phe-ßAla, Phe-Asn, Phe-Gln, Phe-Glu, Phe-Gly, Phe-Nle, Phe-Pyr (besonders Phe-3-Pyr), Phe-Tia (besonders Phe-3-Tia) Tia-His (besonders 3-Tia-His), Trp-His, Tyr-His, Pro-Phe-His oder Pro-Phe-N-Me-His.

Y ist allgemein vorzugsweise -CN oder $-CH_2-NR^{12}-SO_2 R^{14}$, ferner bevorzugt $-CH_2-NR^{12}-CO-NHR^{14}$ oder $-CH_2-NR^{12}-CS-NHR^{14}$.

$R^2$, $R^5$, $R^6$, $R^8$, $R^9$ und $R^{13}$ bedeuten jeweils bevorzugt H, ferner bevorzugt Methyl; $R^5$ ist bevorzugt auch Isopropyl oder Isobutyl. $R^8 R^9 N$ ist bevorzugt auch Pyrrolidino, Piperidino, Morpholino, Amino-piperidino wie 4-Aminopiperidino, Alkylaminopiperidino wie 4-Methylaminopiperidino, Dialkylaminopiperidino wie 4-Dimethylaminopiperidino.

$R^3$ ist vorzugsweise Cycloalkylalkyl, insbesondere Cyclohexylmethyl, ferner bevorzugt Alkyl, insbesondere Isobutyl; Ar-alkyl, insbesondere Benzyl; Cycloalkyl, insbesondere Cyclohexyl.

$R^4$ ist vorzugsweise (H, OH).

$R^7$ ist vorzugssweise A, insbesondere Methyl, Ethyl, Isopropyl oder tert.-Butyl; oder Ar, insbesondere Phenyl, 1- oder 2-Naphthyl.

$R^{10}$ ist vorzugsweise H, Methyl oder CN.

$R^{11}$ ist vorzugsweise H; A, insbesondere Methyl oder tert.-Butyl; $R^8 R^9 N$, insbesondere $A_2 N$ wie $(CH_3)_2 N$, Piperidino oder 4-Aminopiperidino.

$R^{12}$ und $R^{13}$ sind vorzugsweise H oder A, insbesondere Methyl.

$R^{14}$ ist vorzugsweise A, insbesondere Methyl, Ethyl, Propyl, Isopropyl, Butyl, Isobutyl, sek.-Butyl, tert.-Butyl, Pentyl oder Isopentyl; Cycloalkylalkyl, insbesondere Cyclohexylmethyl; Ar-alkyl, insbesondere Benzyl; Ar, insbesondere Phenyl oder Aminophenyl wie p-Aminophenyl.

$R^{15}$ ist vorzugsweise A mit 1-4 C-Atomen, insbesondere Isopropyl oder tert.-Butyl; oder Ar-alkyl, insbesondere Benzyl.

L ist vorzugsweise CH, ferner bevorzugt N.

T ist vorzugsweise O oder NH.

V ist vorzugsweise CO oder $SO_2$.

Der Parameter s ist vorzugsweise 0, aber auch 1; y ist vorzugsweise 1, aber auch 0. Die Summe s + y ist vorzugsweise 1, aber auch 0 oder 2. Der Parameter m ist vorzugsweise 1, 2, 3, 4 oder 5; n ist vorzugsweise 1; p ist vorzugsweise 1; r ist vorzugsweise 1 oder 0. Die Gruppen $C_m H_{2m}$, $C_n H_{2n}$, $C_p H_{2p}$ und $C_r H_{2r}$ sind vorzugsweise geradkettig, bedeuten also vorzugsweise $-(CH_2)_m-$, $-(CH_2)_n-$, $-(CH_2)_p-$ oder $-(CH_2)_r-$.

Dementsprechend bedeutet die Gruppe $R^1$ im einzelnen vorzugsweise $R^8 R^9 N-(CH_2)_m-CO-$, vor allem $H_2 N-C_m H_{2m}-CO-$ wie Aminocarbonyl, Aminoacetyl (H-Gly-), 3-Aminopropionyl (H-ßAla-), 4-Aminobutyryl, 5-Aminopentanoyl, 6-Aminohexanoyl, 7-Aminoheptanoyl, 8-Aminooctanoyl, 9-Aminononanoyl, 10-Aminodecanoyl, 11-Aminoundecanoyl, aber auch z.B. 2-Amino-propionyl (Ala), 2-Amino-2-methylpropionyl; $ANH-C_m H_{2m}-CO-$ wie Methylaminocarbonyl, Methylaminoacetyl (Sarcosyl), 3-Methylaminopropionyl, 4-Methylaminobutyryl, 5-Methylaminopentanoyl, 6-Methylaminohexanoyl, 6-Ethylaminohexanoyl, 7-Methylaminoheptanoyl, 8-Methylaminooctanoyl, 9-Methylaminononanoyl, 10-Methylaminodecanoyl, 11-Methylaminoundeca-noyl; $A_2 N-C_m H_{2m}-CO-$wie Dimethylaminocarbonyl, Dimethylaminoacetyl, 3-Dimethylaminopropionyl, 4-Dimethylaminobutyryl, 5-Dimethylaminopentanoyl, 6-Dimethylaminohexanoyl, 6-Diethylaminohexanoyl, 7-Dimethylaminoheptanoyl, 8-Dimethylaminooctanoyl, 9-Dimethylaminononanoyl, 10-Dimethylaminodecanoyl, 11-Dimethylaminoundecanoyl; $A-O-CO-NH-C_m H_{2m}-CO-$ wie BOC-Gly, ETOC-Gly-, IPOC-Gly, BOC-ßAla, ETOC-ßAla, IPOC-ßAla-, 4-BOC-amino-butyryl, 5-BOC-amino-pentanoyl, 6-BOC-amino-hexanoyl, 7-BOC-amino-heptanoyl, 8-BOC-amino-octanoyl, 9-BOC-amino-nonanoyl, 10-BOC-amino-decanoyl, 11-BOC-amino-unde-canoyl; $ArCH_2-O-CO-NH-C_m H_{2m}-CO-$ wie CBZ-Gly-, CBZ-ßAla, 4-CBZ-amino-butyryl, 5-CBZ-amino-hexanoyl, 7-CBZ-amino-heptanoyl, 8-CBZ-amino-octanoyl, 9-CBZ-amino-nonanoyl, 10-CBZ-amino-decanoyl, 11-CBZ-amino-undecanoyl; $Pyrrolidino-C_m H_{2m}-CO-$ wie Pyrrolidinocarbonyl, Pyrrolidino-acetyl, 3-Pyrrolidino-propionyl, 4-Pyrrolidino-butyryl, 5-Pyrrolidino-pentanoyl, 6-Pyrrolidino-hexanoyl, 4-Pyrrolidino-heptanoyl, 8-Pyrrolidino-octanoyl, 9-Pyrrolidino-nonanoyl, 10-Pyrrolidino-decanoyl; $Piperidino-C_m H_{2m}-CO-$ wie Piperidino-carbonyl, Piperidinoacetyl, 3-Piperidino-propionyl, 4-Piperidino-butyryl, 5-Piperidino-pentanoyl, 6-Piperidino-hexanoyl, 7-Piperidino-heptanoyl, 8-Piperidino-octanoyl, 9-Piperidino-nonanoyl, 10-Piperidino-decanoyl;

Morpholino-$C_mH_{2m}$-CO- wie Morpholinocarbonyl, Morpholinoacetyl, 3-Morpholino-propionyl, 4-Morpholino-butyryl, 5-Morpholino-pentanoyl, 6-Morpholino-hexanoyl, 7-Morpholino-heptanoyl, 8-Morpholino-octanoyl, 9-Morpholino-nonanoyl, 10-Morpholino-decanoyl; 4-Amino-piperidino-$C_mH_{2m}$-CO- wie 4-Amino-piperidino-carbonyl, 4-Amino-piperidino-acetyl, 3-(4-Amino-piperidino)-propionyl, 4-(4-Amino-piperidino)-butyryl, 5-(4-Amino-piperidino)-pentanoyl, 6-(4-Amino-piperidino)-hexanoyl, 7-(4-Amino-piperidino)-heptanoyl, 8-(4-Amino-piperidino)-octanoyl, 9-(4-Amino-piperidino)-nonanoyl, 10-(4-Amino-piperidino)-decanoyl; 4-Dialkylamino-piperidino-$C_mH_{2m}$-CO-wie 4-Dimethylamino-piperidinocarbonyl, 4-Dimethylamino-piperidino-acetyl; 4-Guanidino-piperidino-$C_mH_{2m}$-CO- wie 4-Guanidino-piperidino-carbonyl, 4-Guanidino-piperidino-acetyl; 4-Carboxy-piperidino-$C_mH_{2m}$-CO- wie 4-Carboxy-piperidino-carbonyl, 4-Carboxy-piperidino-acetyl; 4-Alkoxycarbonyl-piperidino-$C_mH_{2m}$-CO- wie 4-Methoxycarbonyl-piperidino-carbonyl, 4-Ethoxycarbonyl-piperidino-carbonyl, 4-Methoxycarbonyl-piperidino-acetyl, 4-Ethoxycarbonyl-piperidino-acetyl; 4-AcNH-piperidino-$C_mH_{2m}$-CO- wie 4-Acetamido-piperidino-carbonyl, 4-Acetamido-piperidino-acetyl; $H_2N$-C(=NH)-NH-$C_mH_{2m}$-CO- wie Guanidinoacetyl, 3-Guanidino-propionyl, 4-Guanidino-butyryl, 5-Guanidino-pentanoyl, 6-Guanidino-hexanoyl, 7-Guanidino-heptanoyl, 8-Guanidino-octanoyl; NC-NH-C(=NH)-NH-$C_mH_{2m}$-CO- wie $N'$-Cyanguanidinoacetyl, 3-($N'$-Cyanguanidino)-propionyl, 4-($N'$-Cyanguanidino)-butyryl, 5-($N'$-Cyanguanidino)-pentanoyl, 6-($N'$-Cyanguanidino)-hexanoyl, 7-($N'$-Cyanguanidino)-heptanoyl, 8-($N'$-Cyanguanidino)-octanoyl; HOOC-$C_mH_{2m}$-CO- wie Malonyl, Succinyl, Glutaryl, Adipyl, 6-Carboxyhexanoyl, 7-Carboxyheptanoyl, 8-Carboxyoctanoyl, 9-Carboxynonanoyl, 10-Carboxy-decanoyl, 11-Carboxyundecanoyl; AOOC-$C_mH_{2m}$-CO- wie Methoxycarbonyl-acetyl, 3-Methoxycarbonyl-propionyl, 4-Methoxycarbonyl-butyryl, 5-Methoxycarbonyl-pentanoyl, 6-Methoxycarbonyl-hexanoyl, 7-Methoxycarbonyl-heptanoyl, 8-Methoxycarbonyl-octanoyl, 9-Methoxycarbonyl-nonanoyl, 10-Methoxycarbonyl-decanoyl, Ethoxycarbonyl-acetyl, 3-Ethoxycarbonyl-propionyl, 4-Ethoxycarbonyl-butyryl, 5-Ethoxycarbonyl-pentanoyl, 6-Ethoxycarbonyl-hexanoyl, 7-Ethoxycarbonyl-heptanoyl, 8-Ethoxycarbonyl-octanoyl, 9-Ethoxycarbonyl-nonanoyl, 10-Ethoxycarbonyl-decanoyl; H-$SO_3$-$C_mH_{2m}$-CO- wie Sulfo-acetyl, 3-Sulfo-propionyl, 4-Sulfo-butyryl, 5-Sulfo-pentanoyl, 6-Sulfo-hexanoyl, 7-Sulfo-heptanoyl, 8-Sulfo-octanoyl, 9-Sulfo-nonanoyl, 10-Sulfo-decanoyl; A-$SO_3$-$C_mH_{2m}$-CO- wie Methoxysulfonyl-acetyl, 3-Methoxysulfonyl-propionyl, 4-Methoxysulfonyl-butyryl, 5-Methoxysulfonyl-pentanoyl, 6-Methoxysulfonyl-hexanoyl, 7-Methoxysulfonyl-heptanoyl, 8-Methoxysulfonyl-octanoyl, 9-Methoxysulfonyl-nonanoyl, 10-Methoxysulfonyl-decanoyl, Ethoxysulfonyl-acetyl, 3-Ethoxysulfonyl-propionyl, 4-Ethoxysulfonyl-butyryl, 5-Ethoxysulfonyl-pentanoyl, 6-Ethoxysulfonyl-hexanoyl, 7-Ethoxysulfonyl-heptanoyl, 8-Ethoxysulfonyl-octanoyl, 9-Ethoxysulfonyl-nonanoyl, 10-Ethoxysulfonyl-decanoyl; $R^{11}$-$C_mH_{2m}$-CO-$C_nH_{2n}$-CH-($R^7$-$C_pH_{2p}$)-$C_rH_{2r}$-CO-, vor allem A-CO-$CH_2$-CH(Ar-$CH_2$)-CO- wie 2-Benzyl-4-oxo-5,5-dimethylhexanoyl, 2-(1-Naphthylmethyl)-4-oxo-5,5-dimethyl-hexanoyl; ferner $R^8R^9$N-CO-$CH_2$-CH(Ar-$CH_2$)-CO-wie 2-Benzyl-3-(4-aminopiperidinocarbonyl)-propionyl, 2-(1-Naphthylmethyl)-3-(4-aminopiperidinocarbonyl)-propionyl; $R^{11}$-$C_mH_{2m}$-$SO_2$-$C_nH_{2n}$-CH($R^7$-$C_pH_{2p}$)-$C_rH_{2r}$-CO-, vor allem A-$SO_2$-$CH_2$-CH(Ar-$CH_2$)-CO wie 2-Benzyl-3-tert.-butylsulfonyl-propionyl,2-(1-naphthylmethyl)-3-tert.-butylsulfonyl-propionyl; $R^{11}$-$C_mH_{2m}$-NH-CO-$C_nH_{2n}$-CH($R^7$-$C_pH_{2p}$)-$C_rH_{2r}$-CO-, vor allem $R^8R^9$N-$(CH_2)_m$-NH-CO-$CH_2$-CH(Ar-$CH_2$)-CO- wie 2-Benzyl-3-(N-3-dimethylaminopropyl-carbamoyl)-propionyl, 2-(1-Naphthylmethyl)-3-(N-3-dimethylaminopropyl-carbamoyl)-propionyl, 2-Benzyl-3-(N-5-dimethylaminopentyl-carbamoyl)-propionyl; A-CH($R^7$-$C_pH_{2p}$)-$C_rH_{2r}$-CO-, vor allem A-CH(Ar-$CH_2$)-CO- wie 2-Benzylhexanoyl, 2-Benzyl-heptanoyl; $R^{11}$-$C_mH_{2m}$-NH-CO-, vor allem $R^8R^9$N-$(CH_2)$-$_m$-NH-CO- wie N-3-Dimethylaminopropyl-carbamoyl, N-5-Dimethylaminopentyl-carbamoyl; $R^{11}$-$C_mH_{2m}$-N($R^7$-$C_pH_{2p}$)-$C_rH_{2r}$-CO-, vor allem A-N(Ar-$CH_2$)-CO- wie N-Benzyl-N-butyl-carbamoyl, N-Benzyl-N-isopentyl-carbamoyl; $R^7$-$C_mH_{2m}$-O-CO-, vor allem A-O-CO-wie ETOC, IPOC, BOC sowie Ar-$C_mH_{2m}$-O-CO- wie CBZ; $R^7$-$C_mH_{2m}$-CO- wie 3,3-Dimethylbutyryl.

Die Gruppe Y bedeutet dementsprechend im einzelnen vorzugsweise -CN; -$CH_2$NH-$SO_2R^{14}$, vor allem -$CH_2$-NH-$SO_2$-A wie Methan-, Ethan-, Propan-, 2-Methylpropan-, Butansulfonamidomethyl, ferner z.B. Phenylmethan-, Cyclohexylmethan-sulfonamidomethyl; -$CH_2$-NH-CO-NH-$R^{14}$, vor allem -$CH_2$-NH-CO-NH-A wie $N'$-Methyl-ureido-methyl, $N'$-Propylureido-methyl, $N'$-Butyl-ureido-methyl, $N'$-Isopentyl-ureido-methyl, ferner -$CH_2$-NH-CO-NH-Ar wie $N'$-Phenyl-ureido-methyl, $N'$-p-Aminophenyl-ureido-methyl; -$CH_2$-NH-CS-NH-$R^{14}$, vor allem -$CH_2$-NH-CS-NH-A wie $N'$-Methyl-thioureido-methyl; -$CH_2$-NH-$COR^{14}$, vor allem -$CH_2$-NH-CO-A wie Acetamidomethyl, Propionamidomethyl, Butyramidomethyl.

Die Verbindungen der Formel I können ein oder mehrere chirale Zentren besitzen und daher in verschiedenen -optisch-aktiven oder optisch-inaktiven - Formen vorkommen. Die Formel I umschließt alle diese Formen. Falls $R^3$ von H verschieden ist und/oder $R^4$ (H, OH) oder (H, $NH_2$) bedeutet, sind die 3S-Hydroxy-, 3S-Amino-, 4S-Amino-, 3S-Hydroxy-4S-amino bzw. 3S,4S-Diamino-Enantiomeren bevorzugt (wobei dem C-Atom, das den Rest $R^4$ trägt, die 3-Stellung, dem, das die Reste $R^1$-Z-$NR^2$ und $R^3$ trägt, die 4-Stellung zugeordnet ist).

Die oben erwähnten Cycloalkyl- und Phenylgruppen sind vorzugsweise unsubstituiert oder tragen vorzugsweise 1 bis 3, insbesondere 1 oder 2 Substituenten.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ik ausgedrückt werden:

Ia $R^7$-$C_mH_{2m}$-O-CO-Z-NH-$CHR^3$-CHOH-$CH_2$-$CHR^5$-Y;

Ib $R^7$-$C_mH_{2m}$-CO-Z-NH-$CHR^3$-CHOH-$CH_2$-$CHR^5$-Y;

Ic $R^8R^9$N-$C_mH_{2m}$-CO-Z-NH-$CHR^3$-CHOH-$CH_2$-$CHR^5$-Y;

Id $R^{10}$-NH-C(=NH)-NH-$C_mH_{2m}$-CO-Z-NH-$CHR^3$-CHOH-$CH_2$-$CHR^5$-Y;

Ie $R^8$OOC-$C_mH_{2m}$-CO-Z-NH-$CHR^3$-CHOH-$CH_2$-$CHR^5$-Y;

If $R^8O_3$S-$C_mH_{2m}$-CO-Z-NH-$CHR^3$-CHOH-$CH_2$-$CHR^5$-Y;

Ig $R^{11}$-$C_mH_{2m}$-$(T)_s$-$(V)_y$-$C_nH_{2n}$ -L($R^7$-$C_pH_{2p}$)-$C_rH_{2r}$-CO-Z-NH-$CHR^3$-CHOH-$CH_2$-$CHR^5$-Y;

Ih 4-Aminopiperidinocarbonyl-Z-NH-$CHR^3$-CHOH-$CH_2$-$CHR^5$-Y;

Ii A-CO-$CH_2$-CH(Ar$CH_2$)-CO-Z-NH-$CHR^3$-CHOH-$CH_2$-$CHR^5$-Y;

Ij A-$SO_2$-$CH_2$-CH(Ar$CH_2$)-CO-Z-NH-$CHR^3$-CHOH-$CH_2$-$CHR^5$-Y;

Ik $R^8R^9$N-$C_mH_{2m}$-NH-CO-$CH_2$-CH(Ar$CH_2$)-CO-Z-NH-$CHR^3$-CHOH-$CH_2$-$CHR^5$-Y.

Insbesondere bevorzugt sind Verbindungen der Teilformeln:

(a) Iaa bis Ika, die den Formeln Ia bis Ik entsprechen, worin jedoch zusätzlich

Z His, Mal-His oder Phe-His bedeutet;

(b) Iab bis Ikb sowie Iaab bis Ikab, die den Formeln Ia bis Ik sowie Iaa bis Ika entsprechen, worin jedoch zusätzlich

$R^3$ Isobutyl oder Cyclohexylmethyl bedeutet;

(c) Iac bis Ikc, Iaac bis Ikac, Iabc bis Ikbc sowie Iaabc bis Ikabc, die den Formeln Ia bis Ik, Iaa bis Ika, Iab bis Ikb sowie Iaab bis Ikab entsprechen, worin jedoch zusätzlich $R^5$ Isopropyl bedeutet.

Insbesondere sind bevorzugt Verbindungen der Teilformeln:

I* sowie Ia* bis Ik*, die den Formeln I sowie Ia bis Ik sowie solche Verbindungen, die den anderen vorstehend genannten Teilformeln entsprechen, worin jedoch zusätzlich

Y -CN oder -$CH_2$-NH-$SO_2$-$R^{14}$ und

$R^{14}$ A, Ar-alkyl oder Cycloalkyl-alkyl bedeuten;

I′ sowie Ia′ bis Ik′, die den Formeln I sowie Ia bis Ik sowie solche Verbindungen, die den anderen vorstehend genannten Teilformeln entsprechen, worin jedoch zusätzlich

Y -$CH_2$-NH-$SO_2$-A bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner EP-A-45665, EP-A-77028, EP-A-77029, EP-A-81783, EP-A-249096) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, z.B. solche die der Formel I entsprechen, aber an Stelle einer His-Gruppe eine N(im)-R′-His-Gruppe (worin R′ eine Aminoschutzgruppe bedeutet, z.B. BOM oder DNP) enthalten, oder solche der Formel $R^1$-Z-$NR^2$-$CHR^3$-CH(NHR′)-$CH_2$-$CR^5R^6$-Y.

Ferner sind Ausgangsstoffe bevorzugt, die an Stelle des H-Atoms einer Hydroxygruppe eine Hydroxyschutzgruppe tragen, z.B. solche der Formel $R^1$-Z-$NR^2$-$CHR^3$-CHOR″-$CH_2$-$CR^5R^6$-Y, worin R″ eine Hydroxyschutzgruppe bedeutet.

Es können auch mehrere - gleiche oder verschiedene -geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. DNP), Aralkoxymethyl- (z.B. BOM) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, ETOC, 2,2,2-Trichlorethoxycarbonyl, IPOC, BOC, 2-Jodethoxycarbonyl; Aralkyloxycarbonyl wie CBZ, 4-Methoxybenzyloxycarbonyl, FMOC. Bevorzugte Aminoschutzgruppen sind BOC, DNP und BOM, ferner CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u. a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z.B. in den genannten Standardwerken und Patentanmeldungen beschrieben sind, z.B. auch nach der Festphasenmethode nach Merrifield.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Dioxan bei 15-30° abgespalten weden, die FMOC-Gruppe mit einer etwa 5-20%igen Lösung von Dimethylamin, Diethylamin oder Piperidin in DMF bei 15-30°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Verbindungen der Formel I können auch durch direkte Peptidsynthese aus einer Carbonsäure- (Formel II) und einer Aminkomponente (Formel III) erhalten werden. Als Carbonsäurekomponenten eignen sich z.B. solche der Teilformeln (a) $R^1$-OH, (b) $R^1$-Z-OH, als Aminkomponenten solche der Teilformeln (a) H-Z-$NR^2$-$CHR^3$-$CR^4$-$CH_2$-$CR^5R^6$-Y, (b) N-$NR^2$-$CHR^3$-$CR^4$-$CH_2$-$CR^5R^6$-Y. Die Peptidbindung kann aber auch innerhalb der Gruppe Z geknüpft werden; dabei wird eine Carbonsäure der Formel $R^1$-$Z^1$-OH mit einer Aminoverbindung der Formel H-$Z^2$-$NR^2$-$CHR^3$-$CR^4$-$CH_2$-$CR^5R^6$-Y umgesetzt, wobei $Z^1$ + $Z^2$ = Z ist. Dabei arbeitet man zweckmäßig nach üblichen Methoden der Peptid-Synthese, wie sie z.B. in Houben-Weyl, l.c., Band 15/II, Seiten 1-806 (1974) beschrieben sind.

Die Reaktion gelingt vorzugsweise in Gegenwart eines Dehydratisierungsmittels, z.B. eines Carbodiimids wie DCCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden, z.B. solche, in denen reaktive Gruppen intermediär durch Schutzgruppen blockiert sind. Die Säurederivate II können z.B. in Form ihrer aktivierten Ester verwendet werden, die zweckmäßig in situ gebildet werden, z.B. durch Zusatz von HOBt oder N-Hydroxysuccinimid.

Harnstoffderivate der Formel I [$R^1$ = $R^8$-NH-CO- oder $R^{11}$-$C_mH_{2m}$-$(T)_s$-$(V)_y$-$C_nH_{2n}$-NH-CO-] sind z.B. erhältlich, indem man ein entsprechendes Isocyanat (z.B. der Formel $R^8$-NCO; herstellbar aus einem Amin der Formel $R^8$-$NH_2$ und Phosgen) mit einem Amin der Formel H-Z-$NR^2$-$CHR^3$-$CR^4$-$CH_2$-$CR^5R^6$-Y (IIa) umsetzt, zweckmäßig in einem inerten Lösungsmittel wie THF bei Temperaturen zwischen etwa -10 und 40°, vorzugsweise zwischen 10 und 30°.

Die Ausgangsstoffe der Formeln II und III sind großenteils bekannt. Sofern sie nicht bekannt sind, können sie nach bekannten Methoden, z.B. den oben angegebenen Methoden der Peptidsynthese und der Abspaltung von Schutzgruppen hergestellt werden.

Gewünschtenfalls kann in einer Verbindung der Formel I eine funktionell abgewandelte Amino- und/oder Hydroxygruppe durch Solvolyse oder Hydrogenolyse nach einer der oben beschriebenen Methoden in Freiheit gesetzt werden.

So kann z. B. eine Verbindung der Formel I, die eine $R^{15}$-$C_xH_{2x}$-O-CO-NH-, eine AcNH-, eine $ArCH_2$-$SO_3$- oder eine AOOC-Gruppe enthält, in die entsprechende Verbindung der Formel I umgewandelt werden, die stattdessen eine $H_2N$-, eine $HSO_3$- oder eine HOOC-Gruppe enthält, zweckmäßig durch selektive Solvolyse nach einer der oben angegebenen Methoden. AOOC-Gruppen können z.B. mit NaOH oder KOH in Wasser-Dioxan bei Temperaturen zwischen 0 und 40°, vorzugsweise 10 und 30°, verseift werden.

Es ist auch möglich, eine Verbindung der Formel I, die eine freie primäre oder sekundäre Aminogruppe enthält, zu acylieren. So kann man insbesondere Verbindungen der Formel I, in denen Y eine $CH_2$-NH-$R^{12}$-Gruppe bedeutet, mit acylierenden Mitteln der Formeln $R^{14}$-$SO_2Cl$, $R^{14}$-COCl oder $R^{14}$-NCO umsetzen, zweckmäßig in Gegenwart eines inerten Lösungsmittels wie THF und/oder einer Base wie Pyridin oder Triethylamin bei Temperaturen zwischen -10 und +30°.

Weiterhin können Ketoverbindungen der Formel I ($R^4$ = O) zu Verbindungen der Formel I ($R^4$ = (H, OH)) reduziert werden, beispielsweise mit einem komplexen Metallhydrid wie $NaBH_4$, das nicht gleichzeitig die Peptid-Carbonylgruppen reduziert, in einem inerten Lösungsmittel wie Methanol bei Temperaturen zwischen etwa -10 und +30°.

Ketoverbindungen der Formel I ($R^4$ = O) können auch durch reduktive Aminierung in Verbindungen der Formel I ($R^4$ = H, $NH_2$) übergeführt werden. Mann kann ein- oder mehrstufig reduktiv aminieren. So kann man z.B. die Ketoverbindung mit Ammoniumsalzen, z.B. Ammoniumacetat, und $NaCNBH_3$ behandeln, vorzugsweise in einem inerten Lösungsmittel, z.B. einem Alkohol wie Methanol, bei Temperaturen zwischen etwa 0 und 50°, insbesondere zwischen 15 und 30°. Weiterhin ist es möglich, die Ketoverbindung zunächst mit Hydroxylamin in üblicher Weise in das Oxim zu überführen und dieses, z.B. durch katalytische Hydrierung an Raney-Nickel, zum Amin zu reduzieren.

Eine Base der Formel I kann mit einer Säure in das zugehörige Säureadditionssalz übergeführt wrden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsauren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff-(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlenhydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-249096 beschriebenen Verbindungen verabreicht, vorzugsweise in Dosierungen zwischen etwa 10 mg und 1 g, insbesondere zwischen 50 und 500 mg pro Dosierungseinheit. Die tägliche Dosierung liegt vorzugsweise zwischen etwa 0,2 und 20 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Renin-abhängige Hypertension und Hyperaldosteronismus können wirksam behandelt werden durch Verabfolgung von Dosierungen zwischen insbesondere etwa 1 und 300, vorzugsweise zwischen 5 und 50 mg/kg Körpergewicht. Für diagnostische Zwecke können die neuen Verbindungen zweckmäßig in Einzeldosen zwischen etwa 0,1 und 10 mg/kg Körpergewicht verabreicht werden.

Vor- und nachstehend sind alle Temperaturen in $^\circ$C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, extrahiert mit Ethylacetat oder Dichlormethan, trennt ab, trocknet die organische Phase über Natriumsulfat, dampft ein und reinigt durch Chromatographie an Kieselgel und/oder Kristallisation.

Beispiel 1

Ein Gemisch von 1038 mg N-[2S-Isopropyl-4S-hydroxy-5S-(4-BOC-aminopiperidinocarbonyl-Phe-(imi-DNP-His)-amino)-6-cyclohexyl-hexyl]-propansulfonsäureamid [erhältlich durch Reaktion von 2,2-Dimethyl-3-BOC-4S-cyclohexylmethyl-5S-iodmethyl-oxazolidin (F. 93$^\circ$) mit Isovaleronitril in Gegenwart von Li-diisopropylamid in THF/Phosphorsäurehexamethyltriamid bei -78$^\circ$ zu 2,2-Dimethyl-3-BOC-4S- cyclohexylmethyl-5S-(2RS-cyan-3-methyl-butyl)-oxazolidin (Öl), chromatographische Trennung der Diastereomeren, Hydrierung des 2S—Epimeren an Raney-Ni in methanolischem NH$_3$ bei 20$^\circ$ zu 2,2-Dimethyl-3-BOC-4S-cyclohexylmethyl-5S-(2S-aminomethyl-3-methyl-butyl)-oxazolidin, Reaktion mit Propansulfonylchlorid/Triethylamin zu 2,2-Dimethyl-3-BOC-4S-cyclohexylmethyl-5S-(2S-propylsulfonylaminomethyl-3-methyl-butyl)-oxazolidin, Spaltung mit 4 n HCl in Dioxan/Methanol zu N-(2S-Isopropyl-4S-hydroxy-5S-amino-6-cyclohexyl-hexyl)-propansulfonsäureamid (Hydrochlorid, F. 61-63$^\circ$), Kondensation mit BOC-(imi-DNP-His)-OH zu N-(2S-Isopropyl-4S-hydroxy-5S-BOC-(imi-DNP-His)-amino-6-cyclohexyl-hexyl)-propansulfonsäureamid (F. 68$^\circ$), Abspaltung der BOC-Gruppe mit HCl/Dioxan und Kondensation mit 4-BOC-amino-piperidinocarbonyl-Phe-OH (F. 158-159$^\circ$)], 2 g 2-Mercaptoethanol, 20 ml DMF und 20 ml Wasser wird unter Rühren bei 20$^\circ$ mit wäßriger Na$_2$CO$_3$-Lösung auf pH 8 eingestellt und 2 Std. bei 20$^\circ$ gerührt. Nach üblicher Aufarbeitung erhält man N-[2S-Isopropyl-4S-hydroxy-5S-(4-BOC-amino-piperidinocarbonyl-Phe-His-amino)-6-cyclohexylhexyl]-propansulfonsäureamid, F. 121$^\circ$.

Analog erhält man aus den entsprechenden imi-DNP-His-Derivaten:

2S-Isopropyl-4S-hydroxy-5S-(2-benzyl-4-oxo-5,5-dimethyl-hexanoyl-His-amino)-6-cyclohexyl-hexansäurenitril, F. 129° (Zers.)

N-[2S-Isopropyl-4S-hydroxy-5S-(2-benzyl-3-tert.-butylsulfonyl-propionyl-His-amino)-6-cyclohexyl-hexyl]-methansulfonsäureamid, 2 Epimere, F. 116° (Hydrochlorid, F. 112°) bzw. F. 122° (Hydrochlorid, F. 107°) [über N-[2S-Isopropyl-4S-hydroxy-5S-amino-6-cyclohexyl-hexyl]- methansulfonsäureamid (Hydrochlorid, F. 78°) und N-(2S-Isopropyl-4S-hydroxy-5S-BOC-(imi-DNP-His)-amino-6-cyclohexyl-hexyl)-methansulfonsäureamid (F. 120°)];

N-[2S-Isopropyl-4S-hydroxy-5S-(2-benzyl-3-tert.-butylsulfonyl-propionyl-His-amino)-6-cyclohexyl-hexyl]-propansulfonsäureamid, 2 Epimere, F. 70° (Hydrochlorid, F. 99-100°) bzw. F. 103° (Hydrochlorid, F. 104-105°);

N-[2S-Isopropyl-4S-hydroxy-5S-(2-benzyl-3-tert.-butylsulfonyl-propionyl-His-amino)-6-cyclohexyl-hexyl]-butansulfonsäureamid, 2 Epimere, F. 79° (Hydrochlorid, F. 109°) bzw. F. 97° (Hydrochlorid, F. 122°) | über N-(2S-Isopropyl-4S-hydroxy-5S-amino-6-cyclohexyl-hexyl)-butansulfonsäureamid (Hydrochlorid, F. 56°) und N-(2S-Isopropyl-4S-hydroxy-5S-BOC-(imi-DNP-His)-amino-6-cyclohexyl-hexyl)-butansulfonsäureamid (F. 90°)];

N-[2S-Isopropyl-4S-hydroxy-5S-(2-benzyl-heptanoyl-His-amino)-6-cyclohexyl-hexyl]-propansulfonsäureamid

N-[2S-Isopropyl-4S-hydroxy-5S-(2-benzyl-4-oxo-5,5-dimethyl-hexanoyl-His-amino)-6-cyclohexyl-hexyl]-propansulfonsäureamid

N-[2S-Isopropyl-4S-hydroxy-5S-(2-benzyl-3-tert.-butylsulfonyl-propionyl-His-amino)-6-cyclohexyl-hexyl]-propansulfonsäureamid

N-[2S-Isopropyl-4S-hydroxy-5S-(2-benzyl-3-morpholinocarbonyl-propionyl-His-amino)-6-cyclohexyl-hexyl]-propansulfonsäureamid

N-[2S-Isopropyl-4S-hydroxy-5S-(2-(1-naphthylmethyl)-3-morpholinocarbonyl-propionyl-His-amino)-6-cyclohexyl-hexyl]-propansulfonsäureamid

N-[2S-Isopropyl-4S-hydroxy-5S-(N-benzyl-N-isopentyl-carbamoyl-His-amino)-6-cyclohexyl-hexyl]-propansulfonsäureamid

N-[2S-Isopropyl-4S-hydroxy-5S-(2-(1-naphthylmethyl)-3-(N-(3-dimethylaminopropyl)-carbamoyl-propionyl-His-amino)-6-cyclohexyl-hexyl]-propansulfonsäureamid

N-[2S-Isopropyl-4S-hydroxy-5S-(2-benzyl-3-(4-BOC-amino-piperidinocarbonyl)-propionyl-His-amino)-6-cyclohexyl-hexyl]-propansulfonsäureamid

N-[2S-Isopropyl-4S-hydroxy-5S-(2-(1-naphthylmethyl)-3-(4-BOC-amino-piperidinocarbonyl)-propionyl-His-amino)-6-cyclohexyl-hexyl]-propansulfonsäureamid

N-[2S-Isopropyl-4S-hydroxy-5S-(BOC-Phe-His-amino)-6-cyclohexyl-hexyl]-propansulfonsäureamid

N-[2S-Isopropyl-4S-hydroxy-5S-(CBZ-Phe-His-amino)-6-cyclohexyl-hexyl]-propansulfonsäureamid

N-[2S-Isopropyl-4S-hydroxy-5S-(3,3-dimethylbutyryl-Phe-His-amino)-6-cyclohexyl-hexyl]-propansulfonsäureamid

N-[2S-Isopropyl-4S-hydroxy-5S-(2-BOC-amino-2-methylpropionyl-Phe-His-amino)-6-cyclohexyl-hexyl]-propansulfonsäureamid

N-[2S-Isopropyl-4S-hydroxy-5S-(6-BOC-amino-hexanoyl-Phe-His-amino)-6-cyclohexyl-hexyl]-propansulfonsäureamid

N-[2S-Isopropyl-4S-hydroxy-5S-(4-BOC-amino-piperidinocarbonyl-Phe-His-amino)-6-cyclohexyl-hexyl]-propansulfonsäureamid

N-[2S-Isopropyl-4S-hydroxy-5S-(6-Methoxycarbonyl-hexanoyl-Phe-His-amino)-6-cyclohexyl-hexyl]-propansulfonsäureamid

N-[2S-Isopropyl-4S-hydroxy-5S-(N-(3-dimethylaminopropyl)carbamoyl-Phe-His-amino)-6-cyclohexyl-hexyl]-propansulfonsäureamid

N-[2S-Isopropyl-4S-hydroxy-5S-(N-(5-dimethylaminopentyl)carbamoyl-Phe-His-amino)-6-cyclohexyl-hexyl]-propansulfonsäureamid

N-[2S-Isopropyl-4S-hydroxy-5S-(BOC-Mal-His-amino)-6-cyclohexyl-hexyl]-propansulfonsäureamid

N-[2S-Isopropyl-4S-hydroxy-5S-(CBZ-Mal-His-amino)-6-cyclohexyl-hexyl]-propansulfonsäureamid

N-[2S-Isopropyl-4S-hydroxy-5S-(3,3-dimethylbutyryl-Mal-His-amino)-6-cyclohexyl-hexyl]-propansulfonsäureamid

N-[2S-Isopropyl-4S-hydroxy-5S-(2-BOC-amino-2-methylpropionyl-Mal-His-amino)-6-cyclohexyl-hexyl]-propansulfonsäureamid

N-[2S-Isopropyl-4S-hydroxy-5S-(6-BOC-amino-hexanoyl-Mal-His-amino)-6-cyclohexyl-hexyl]-propansulfonsäureamid

N-[2S-Isopropyl-4S-hydroxy-5S-(4-BOC-amino-piperidinocarbonyl-Mal-His-amino)-6-cyclohexyl-hexyl]-propansulfonsäureamid

N-[2S-Isopropyl-4S-hydroxy-5S-(6-Methoxycarbonyl-hexanoyl-Mal-His-amino)-6-cyclohexyl-hexyl]-propansulfonsäureamid

N-[2S-Isopropyl-4S-hydroxy-5S-(N-(3-dimethylaminopropyl)carbamoyl-Mal-His-amino)-6-cyclohexyl-hexyl]-propansulfonsäureamid

N-[2S-Isopropyl-4S-hydroxy-5S-(N-(5-dimethylaminopentyl)-carbamoyl-Mal-His-amino)-6-cyclohexyl-hexyl]-propansulfonsäureamid

N-[4S-Hydroxy-5S-(4-BOC-aminopiperidinocarbonyl-Phe-His-amino)-6-cyclohexyl-hexyl]-propansulfonsäureamid

N-[4S-Hydroxy-5S-(4-BOC-aminopiperidinocarbonyl-Mal-His-amino)-6-cyclohexyl-hexyl]-propansulfonsäureamid

N-[2S-Methyl-4S-hydroxy-5S-(4-BOC-aminopiperidinocarbonyl-Phe-His-amino)-6-cyclohexyl-hexyl]-propansulfonsäureamid

N-[2S-Methyl-4S-hydroxy-5S-(4-BOC-aminopiperidinocarbonyl-Mal-His-amino)-6-cyclohexyl-hexyl]-propansulfonsäureamid

N-[2S-Isobutyl-4S-hydroxy-5S-(4-BOC-aminopiperidinocarbonyl-Phe-His-amino)-6-cyclohexyl-hexyl]-methansulfonsäureamid

N-[2S-Isobutyl-4S-hydroxy-5S-(4-BOC-aminopiperidinocarbonyl-Mal-His-amino)-6-cyclohexyl-hexyl]-methansulfonsäureamid

N-[4S-Hydroxy-5S-(2-(1-naphthylmethyl)-3-(4-BOC-aminopiperidinocarbonyl)-propionyl-His-amino)-6-cyclohexyl-hexyl]-propansulfonsäureamid

N-[2S-Methyl-4S-hydroxy-5S-(2-(1-naphthylmethyl)-3-(4-BOC-aminopiperidinocarbonyl)-propionyl-His-amino)-6-cyclohexyl-hexyl]-propansulfonsäureamid

N-[2S-Isopropyl-4S-hydroxy-5S-(2-(1-naphthylmethyl)-3-(4-BOC-aminopiperidinocarbonyl)-propionyl-His-amino)-6-cyclohexyl-hexyl]-propansulfonsäureamid

N-[4S-Hydroxy-5S-(2-benzyl-3-tert.-butylsulfonyl-propionyl-His-amino)-6-cyclohexyl-hexyl]-propansulfonsäureamid

N-[2S-Methyl-4S-hydroxy-5S-(2-benzyl-3-tert.-butylsulfonyl-propionyl-His-amino)-6-cyclohexyl-hexyl]-propansulfonsäureamid

N-[2S-Isopropyl-4S-hydroxy-5S-(BOC-Phe-His-amino)-6-cyclohexyl-hexyl]-2-methylpropansulfonsäureamid

N-[2S-Isopropyl-4S-hydroxy-5S-(BOC-Cal-His-amino)-6-cyclohexyl-hexyl]-2-methylpropansulfonsäureamid

N-[2S-Isopropyl-4S-hydroxy-5S-(BOC-αNal-His-amino)-6-cyclohexyl-hexyl]-2-methylpropansulfonsäureamid

N-[2S-Isopropyl-4S-hydroxy-5S-(BOC-2-Tia-His-amino)-6-cyclohexyl-hexyl]-2-methylpropansulfonsäureamid

N-[2S-Isopropyl-4S-hydroxy-5S-(BOC-Trp-His-amino)-6-cyclohexyl-hexyl]-2-methylpropansulfonsäureamid

N-[2S-Isopropyl-4S-hydroxy-5S-(BOC-Pro-Phe-His-amino)-6-cyclohexyl-hexyl]-2-methylpropansulfonsäureamid

N-[2S-Isopropyl-4S-hydroxy-5S-(3,3-dimethylbutyryl-Pro-Phe-N-Me-His-amino)-6-cyclohexyl-hexyl]-2-methylpropansulfonsäureamid

N-[2S-Isopropyl-4S-hydroxy-5S-(4-CBZ-amino-piperidinocarbonyl-Phe-His-amino)-6-cyclohexyl-hexyl]-propansulfonsäureamid.

Beispiel 2

Man löst 1 g N-[2S-Isopropyl-4S-hydroxy-5S-(3-(N-3-dimethylaminopropyl-carbamoyl)-2-(1-naphthylmethyl)-propionyl-(imi-BOM-His)-amino)-6-cyclohexyl-hexyl]-propansulfonsäureamid [Stereoisomerengemisch; erhältlich durch Kondensation von N-(2S-Isopropyl-4S-hydroxy-5S-amino-6-cyclohexyl-hexyl)-propansulfonsäureamid mit BOC-(imi-BOM-His)-OH zu N-[2S-Isopropyl-4S-hydroxy-5S-BOC-(imi-BOM-His)-amino-6-cyclohexyl-hexyl)-propansulfonsäureamid, Abspaltung der BOC-Gruppe und Kondensation mit DL-3-(N-3-Dimethylaminopropyl-carbamoyl)-2-(1-naphthylmethyl)-propionsäure, F. 122-123°] in 25 ml Ethanol, hydriert an 0,5 g 10%ig. Pd-C bei 20° und 1 bar bis zum Stillstand der $H_2$-Aufnahme, filtriert, dampft ein und erhält nach chromatographischer Reinigung N-[2S-Isopropyl-4S-hydroxy-5S-(3-(N-3-dimethylaminopropyl-carbamoyl)-2-(1-naphthylmethyl)-propionyl-His-amino)-6-cyclohexyl-hexyl]-propansulfonsäureamid, 2 Epimere, F. 102° (Hydrochlorid, F. 119°) bzw. Öl (Hydrochlorid, F. 117°).

Analog erhält man durch Hydrogenolyse der entsprechenden imi-BOM-His-Derivate

N-[2S-Isopropyl-4S-hydroxy-5S-(2S-benzyl-3-tert.-butylsulfonyl-propionyl-His-amino)-6-cyclohexyl-hexyl]-methansulfonsäureamid, F. 103°; Hydrochlorid, F. 104-105°

N-[2S-Isopropyl-4S-hydroxy-5S-(2S-benzyl-3-tert.-butylsulfonyl-propionyl-His-amino)-6-cyclohexyl-hexyl]-propansulfonsäureamid, F. 122°; Hydrochlorid, F. 122°

14

N-[2S-Isopropyl-4S-hydroxy-5S-(2S-benzyl-3-tert.-butylsulfonyl-propionyl-His-amino)-6-cyclohexyl-hexyl]-butansulfonsäureamid, F. 97° ; Hydrochlorid, F. 122°
N-[2S-Isopropyl-4S-hydroxy-5S-(2S-benzyl-3-tert.-butylsulfonyl-propionyl-His-amino)-6-cyclohexyl-hexyl]-2-methyl-propansulfonsäureamid
N-[2S-Isopropyl-4S-hydroxy-5S-(2S-benzyl-3-tert.-butylsulfonyl-propionyl-His-amino)-6-cyclohexyl-hexyl]-phenylmethan-sulfonsäureamid
N-[2S-Isopropyl-4S-hydroxy-5S-(2S-benzyl-3-tert.-butylsulfonyl-propionyl-His-amino)-6-cyclohexyl-hexyl]-cyclohexylmethan-sulfonsäureamid
N-[2S-Isopropyl-4S-hydroxy-5S-(2S-benzyl-3-tert.-butylsulfonyl-propionyl-His-amino)-6-cyclohexyl-hexyl]-N′-propyl-harnstoff
N-[2S-Isopropyl-4S-hydroxy-5S-(2S-benzyl-3-tert.-butylsulfonyl-propionyl-His-amino)-6-cyclohexyl-hexyl]-N′-butyl-harnstoff
N-[2S-Isopropyl-4S-hydroxy-5S-(2S-benzyl-3-tert.-butylsulfonyl-propionyl-His-amino)-6-cyclohexyl-hexyl]-N′-propyl-thioharnstoff
N-[2S-Isopropyl-4S-hydroxy-5S-(2S-benzyl-3-tert.-butylsulfonyl-propionyl-His-amino)-6-cyclohexyl-hexyl]-N′-(p-aminophenyl)-harnstoff
N-[2S-Isopropyl-4S-hydroxy-5S-(2S-benzyl-3-tert.-butylsulfonyl-propionyl-His-amino)-6-cyclohexyl-hexyl]-4-methylpentansäureamid.

## Beispiel 3

Analog Beispiel 2 erhält man durch Hydrogenolyse von 1-CBZ-amino-4S-hydroxy-5S-(BOC-Phe-Gly-amino)-6-cyclohexyl-hexan (erhältlich durch Reaktion von 1-CBZ-amino-4S-hydroxy-5S-amino-6-cyclohexyl-hexan mit BOC-Phe-Gly-OH) das 4S-Hydroxy-5S-(BOC-Phe-Gly-amino)-6-cyclohexyl-hexylamin.
Analog erhält man:
2S-Methyl-4S-hydroxy-5S-(BOC-Phe-Gly-amino)-6-cyclohexyl-hexylamin
2S-Ethyl-4S-hydroxy-5S-(BOC-Phe-Gly-amino)-6-cyclohexyl-hexylamin
2S-Isopropyl-4S-hydroxy-5S-(BOC-Phe-Gly-amino)-6-cyclohexyl-hexylamin
2S-Isobutyl-4S-hydroxy-5S-(BOC-Phe-Gly-amino)-6-cyclohexyl-hexylamin.
Analog erhält man durch Hydrogenolyse von N-[2S-Isopropyl-4S-hydroxy-5S-(3-benzyloxysulfonyl-propionyl-Phe-His-amino)-7-methyl-octyl]-propansulfonsäureamid das N-[2S-Isopropyl-4S-hydroxy-5S-(3-sulfopropionyl-Phe-His-amino)-7-methyl-octyl]-propansulfonsäureamid.

## Beispiel 4

Eine Lösung von 4,33 g N-[2S-Isopropyl-4S-hydroxy-5S-(H-ßAla-amino)-6-cyclohexyl-hexyl]-propansulfonsäureamid [erhältlich durch Kondensation von BOC-ßAla-OH mit N-(2S-Isopropyl-4S-hydroxy-5S-amino-6-cyclohexyl-hexyl)-propansulfonsäureamid zu N-[2S-Isopropyl-4S-hydroxy-5S-(BOC-ßAla-amino)-6-cyclohexyl-hexyl]-propansulfonsäureamid und Abspaltung der BOC-Gruppe mit 4 n HCl in Dioxan] in 60 ml Dichlormethan wird mit 1,01 g N-Methylmorpholin versetzt. Unter Rühren gibt man 3,19 g 4-Dimethylamino-piperidinocarbonyl-Phe-OH, 1,35 g HOBt und eine Lösung von 2.06 g DCCI in 50 ml Dichlormethan hinzu, rührt 14 Std. bei 2-6°, filtriert den ausgeschiedenen Dicyclohexylharnstoff ab und dampft das Filtrat ein. Nach üblicher Aufarbeitung erhält man N-[2S-Isopropyl-4S-hydroxy-5S-(4-dimethylamino-piperidinocarbonyl-Phe-ßAla-amino)-6-cyclohexyl-hexyl]-propansulfonsäureamid.
Analog erhält man mit 4-BOC-amino-piperidinocarbonyl-Phe-OH das N-[2S-Isopropyl-4S-hydroxy-5S-(4-BOC-amino-piperidinocarbonyl-Phe-ßAla-amino)-6-cyclohexyl-hexyl]-propansulfonsäureamid.
Analog erhält man:
N-[2S-Isopropyl-4S-hydroxy-5S-(4-BOC-amino-piperidinocarbonyl-Phe-2-Tia-amino)-6-cyclohexyl-hexyl]-propansulfonsäureamid
N-[2S-Isopropyl-4S-hydroxy-5S-(N-(5-dimethylaminopentyl)-carbamoyl-Phe-ßAla-amino)-6-cyclohexyl-hexyl]-propansulfonsäureamid
N-[2S-Isopropyl-4S-hydroxy-5S-(N-(5-dimethylaminopentyl)-carbamoyl-Phe-2-Tia-amino)-6-cyclohexyl-hexyl]-propansulfonsäureamid
N-[4S-Hydroxy-5S-(BOC-Phe-Gly-amino)-7-methyl-octyl]-piperidin.

Beispiel 5

Analog Beispiel 4 erhält man aus BOC-Phe-Gly-OH und N-(2S-Isopropyl-4S-hydroxy-5S-amino-6-cyclohexyl-hexyl)-propansulfonsäureamid das N-(2S-Isopropyl-4S-hydroxy-5S-BOC-Phe-Gly-amino-6-cyclohexyl-hexyl)-propansulfonsäureamid.

Analog erhält man
N-[2S-Isopropyl-4S-hydroxy-5S-BOC-Phe-Abu-amino-6-cyclohexyl-hexyl)-propansulfonsäureamid
N-[2S-Isopropyl-4S-hydroxy-5S-BOC-Phe-Ala-amino-6-cyclohexyl-hexyl)-propansulfonsäureamid
N-[2S-Isopropyl-4S-hydroxy-5S-BOC-Phe-ßAla-amino-6-cyclohexyl-hexyl)-propansulfonsäureamid
N-[2S-Isopropyl-4S-hydroxy-5S-BOC-Phe-Asn-amino-6-cyclohexyl-hexyl)-propansulfonsäureamid
N-[2S-Isopropyl-4S-hydroxy-5S-BOC-Phe-Gln-amino-6-cyclohexyl-hexyl)-propansulfonsäureamid
N-[2S-Isopropyl-4S-hydroxy-5S-BOC-Phe-Nle-amino-6-cyclohexyl-hexyl)-propansulfonsäureamid
N-[2S-Isopropyl-4S-hydroxy-5S-BOC-Phe-3-Pya-amino-6-cyclohexyl-hexyl)-propansulfonsäureamid
N-[2S-Isopropyl-4S-hydroxy-5S-BOC-Phe-2-Tia-amino-6-cyclohexyl-hexyl)-propansulfonsäureamid
N-[3S-Hydroxy-4S-(4-ethoxycarbonyl-piperidino-carbonyl-Phe-Gly-amino)-5-cyclohexyl-pentyl]-propansulfonsäureamid.

Beispiel 6

Analog Beispiel 4 erhält man aus Pivalinsäure und N-[2S-Isopropyl-4S-hydroxy-5S-(H-Phe-Gly-amino)-6-cyclohexyl-hexyl]-propansulfonsäureamid das N-[2S-Isopropyl-4S-hydroxy-5S-(pivaloyl-Phe-Gly-amino)-6-cyclohexyl-hexyl]-propansulfonsäureamid.

Beispiel 7

Analog Beispiel 4 erhält man aus 4-BOC-aminopiperidinocarbonyl-Phe-OH und N-[2S-Isopropyl-4S-hydroxy-5S-(H-Gly-amino)-7-methyl-octyl]-ethansulfonsäureamid das N-[2S-Isopropyl-4S-hydroxy-5S-(4-BOC-aminopiperidinocarbonyl-Phe-Gly-amino)-7-methyl-octyl]-ethansulfonsäureamid.

Beispiel 8

Eine Lösung von 1,25 g Cyclohexylisocyanat in 15 ml THF wird unter Rühren bei 20° zugetropft zu einer Lösung von 5,6 g 2S-Isopropyl-4S-hydroxy-5S-(BOC-Phe-Gly-amino)-6-cyclohexyl-hexylamin in 60 ml THF. Man rührt noch 3 Std. bei 20°, arbeitet wie üblich auf und erhält N-[2S-Isopropyl-4S-hydroxy-5S-(BOC-Phe-Gly-amino)-6-cyclohexyl-hexyl]-N'-cyclohexyl-harnstoff.

Beispiel 9

Eine Lösung von 1 g N-[2S-Isopropyl-4S-hydroxy-5S-(4-BOC-amino-piperidinocarbonyl-Phe-His-amino)-6-cyclohexyl-hexyl]-propansulfonsäureamid in 20 ml 4 n HCl in Dioxan wird 30 Min. bei 20° gerührt und dann eingedampft. Man erhält N-[2S-Isopropyl-4S-hydroxy-5S-(4-amino-piperidinocarbonyl-Phe-His-amino)-6-cyclohexyl-hexyl]-propansulfonsäureamid, Dihydrochlorid, F. 161-162°.

Analog erhält man durch Spaltung der entsprechenden BOC-Derivate:
N-[2S-Isopropyl-4S-hydroxy-5S-(2-benzyl-3-(4-aminopiperidinocarbonyl)-propionyl-His-amino)-6-cyclohexyl-hexyl]-propansulfonsäureamid
N-[2S-Isopropyl-4S-hydroxy-5S-2-(1-naphthylmethyl)-3-(4-aminopiperidinocarbonyl)-propionyl-His-amino)-6-cyclohexyl-hexyl]-propansulfonsäureamid
N-[2S-Isopropyl-4S-hydroxy-5S-(4-aminopiperidinocarbonyl-Phe-ßAla-amino)-6-cyclohexyl-hexyl]-propansulfonsäureamid
N-[2S-Isopropyl-4S-hydroxy-5S-(4-aminopiperidinocarbonyl-Phe-2-Tia-amino)-6-cyclohexyl-hexyl]-propansulfonsäureamid
N-[2S-Isopropyl-4S-hydroxy-5S-(4-aminopiperidinocarbonyl-Phe-His-amino)-6-cyclohexyl-hexyl]-propansulfonsäureamid
N-[2S-Isopropyl-4S-hydroxy-5S-(4-aminopiperidinocarbonyl-Mal-His-amino)-6-cyclohexyl-hexyl]-

propansulfonsäureamid
N-[2S-Isopropyl-4S-hydroxy-5S-(2-amino-2-methyl-propionyl-Phe-His-amino)-6-cyclohexyl-hexyl]-propansulfonsäureamid
N-[2S-Isopropyl-4S-hydroxy-5S-(2-amino-2-methyl-propionyl-Mal-His-amino)-6-cyclohexyl-hexyl]-propansulfonsäureamid
N-[2S-Isopropyl-4S-hydroxy-5S-(6-aminohexanoyl-Phe-His-amino)-6-cyclohexyl-hexyl]-propansulfonsäureamid
N-[2S-Isopropyl-4S-hydroxy-5S-(6-aminohexanoyl-Mal-His-amino)-6-cyclohexyl-hexyl]-propansulfonsäureamid
N-[4S-Hydroxy-5S-(4-aminopiperidinocarbonyl-Phe-His-amino)-6-cyclohexyl-hexyl]-propansulfonsäureamid, Dihydrochlorid, F. 125° (Zers.)
N-[4S-Hydroxy-5S-(4-aminopiperidinocarbonyl-Mal-His-amino)-6-cyclohexyl-hexyl]-propansulfonsäureamid
N-[2S-Methyl-4S-hydroxy-5S-(4-aminopiperidinocarbonyl-Phe-His-amino)-6-cyclohexyl-hexyl]-propansulfonsäureamid
N-[2S-Methyl-4S-hydroxy-5S-(4-aminopiperidinocarbonyl-Mal-His-amino)-6-cyclohexyl-hexyl]-propansulfonsäureamid
N-[2S-Isobutyl-4S-hydroxy-5S-(4-aminopiperidinocarbonyl-Phe-His-amino)-6-cyclohexyl-hexyl]-methansulfonsäureamid
N-[2S-Isobutyl-4S-hydroxy-5S-(4-aminopiperidinocarbonyl-Mal-His-amino)-6-cyclohexyl-hexyl]-methansulfonsäureamid
N-[4S-Hydroxy-5S-(2-(1-naphthylmethyl)-3-(4-aminopiperidinocarbonyl)-propionyl-His-amino)-6-cyclohexyl-hexyl]-propansulfonsäureamid
N-[2S-Methyl-4S-hydroxy-5S-(2-(1-naphthylmethyl)-3-(4-aminopiperidinocarbonyl)-propionyl-His-amino)-6-cyclohexyl-hexyl]-propansulfonsäureamid
N-[2S-Isopropyl-4S-hydroxy-5S-(2-(1-naphthylmethyl)-3-(4-aminopiperidinocarbonyl)-propionyl-His-amino)-6-cyclohexyl-hexyl]-propansulfonsäureamid.

Beispiel 10

Ein Gemisch von 1 g N-[3S-Hydroxy-4S-(4-ethoxycarbonylpiperidino-carbonyl-Phe-gly-amino)-5-cyclohexyl-pentyl]-propansulfonsäureamid, 50 ml Dioxan und 20 ml 2 n NaOH (wäßrig) wird 3 Std. bei 20° gerührt. Nach üblicher Aufarbeitung erhält man N-[3S-Hydroxy-4S-(4-carboxy-piperidinocarbonyl-Phe-Gly-amino)-5-cyclohexyl-pentyl]-propansulfonsäureamid.

Analog erhält man durch Verseifung der entsprechenden Methylester:
N-[2S-Isopropyl-4S-hydroxy-5S-(6-carboxyhexanoyl-Phe-His-amino)-6-cyclohexyl-hexyl]-propansulfonsäureamid
N-[2S-Isopropyl-4S-hydroxy-5S-(6-carboxyhexanoyl-Mal-His-amino)-6-cyclohexyl-hexyl]-propansulfonsäureamid.

Beispiel 11

Eine Lösung von 5,18 g 4S-Hydroxy-5S-(BOC-Phe-Gly-amino)-6-cyclohexyl-hexylamin in 250 ml THF wird mit 1,01 g Triethylamin und dann unter Rühren bei 0° tropfenweise mit einer Lösung von 1,15 g Methansulfonylchlorid in 10 ml THF versetzt. Man rührt noch 1 Std., wobei man die Temperatur auf 20° steigen läßt, arbeitet wie üblich auf und erhält N-[4S-Hydroxy-5S-(BOC-Phe-Gly-amino)-6-cyclohexyl-hexyl]-methansulfonsäureamid.

Beispiel 12

Analog Beispiel 3 erhält man aus N-[2S-Isopropyl-4S-hydroxy-5S-(4-CBZ-amino-piperidinocarbonyl-Phe-His-amino)-6-cyclohexyl-hexyl]-propansulfonsäureamid durch Hydrogenolyse das N-[2S-Isopropyl-4S-hydroxy-5S-(4-amino-piperidinocarbonyl-Phe-His-amino)-6-cyclohexyl-hexyl]-propansulfonsäureamid,     F. 161-162°.

Beispiel 13

a) Analog Beispiel 4 erhält man aus 4-Dimethylaminobutyryl-Phe-Gly-OH und N-(2S-Isopropyl-4-oxo-5S-amino-6-cyclohexyl-hexyl)-propansulfonsäureamid     das     N-[2S-Isopropyl-4-oxo-5S-(4-dimethylaminobutyryl-Phe-Gly-amino)-6-cyclohexyl-hexyl]-propansulfonsäureamid.

b) Eine Lösung von 1 g des vorstehenden Keto-amids in 25 ml CH$_3$OH wird an 0,1 g 10%ig. Pd-C bei 20° und 1 bar bis zum Stillstand der H$_2$-Aufnahme hydriert. Nach Filtrieren und Eindampfen erhält man ein Gemisch von N-[2S-Isopropyl-4R- und -4S-hydroxy-5S-(4-dimethylaminobutyryl-Phe-Gly-amino)-6-cyclohexyl-hexyl]-propansulfonsäureamid.

Beispiel 14

Eine Lösung von 627 mg des nach Beispiel 13a) erhältlichen Keto-amids und 1,43 g Na$_2$CO$_3$ . 10 H$_2$O in 5 ml Methanol und 5 ml Wasser wird mit 70 mg Hydroxylaminhydrochlorid versetzt und 14 Std. bei 20° gerührt. Das ausgefallene Oxim wird abfiltriert, getrocknet, in 10 ml Methanol gelöst und an 0,5 g Raney-Ni bei 20° und 5 bar hydriert. Man filtriert den Katalysator ab, dampft das Filtrat ein, trennt das erhaltene Gemisch an Kieselgel und erhält N-[2S-Isopropyl-4S-amino-5S-(4-dimethylaminobutyryl-Phe-Gly-amino)-6-cyclohexyl-hexyl]-propansulfonsäureamid; daneben erhält man das 4R-Amino-Epimere.

Beispiel 15

Analog Beispiel 2 erhält man aus den entsprechenden imi-BOM-His-Derivaten:
N-[4S-Hydroxy-5S-(2S-benzyl-3-tert.-butylsulfonyl-propionyl-His-amino)-6-cyclohexyl-hexyl]-propansulfonsäureamid, F. 90-91°
N-[4S-Hydroxy-5S-(2R-benzyl-3-tert.-butylsulfonyl-propionyl-His-amino)-6-cyclohexyl-hexyl]-propansulfonsäureamid, F. 81-82°
N-[4S-Hydroxy-5S-(4 BOC-amino-piperidinocarbonyl-Phe-His-amino)-6-cyclohexyl-hexyl]-propansulfonsäureamid, F. 118-120°
N-[4S-Hydroxy-5S-(4-BOC-amino-piperidinocarbonyl-Mal-His-amino)-6-cyclohexyl-hexyl]-propansulfonsäureamid, F. 123-125°
N-[2R-Methyl-4S-hydroxy-5S-(4-BOC-amino-piperidinocarbonyl-Phe-His-amino)-6-cyclohexyl-hexyl]-propansulfonsäureamid, F. 164° (Zers.)
N-[2S-Methyl-4S-hydroxy-5S-(4-BOC-amino-piperidinocarbonyl-Phe-His-amino)-6-cyclohexyl-hexyl]-propansulfonsäureamid, F. 140° (Zers.)
N-[2R-Methyl-4S-hydroxy-5S-(4-BOC-amino-piperidinocarbonyl-Mal-His-amino)-6-cyclohexyl-hexyl]-propansulfonsäureamid, F. 133° (Zers.)
N-[2R-Methyl-4S-hydroxy-5S-(2R-benzyl-3-tert.-butylsulfonyl-propionyl-His-amino)-6-cyclohexyl-hexyl]-propansulfonsäureamid, Hydrochlorid, F. 124° (Zers.)
N-[2R-Methyl-4S-hydroxy-5S-(2S-benzyl-3-tert.-butylsulfonyl-propionyl-His-amino)-6-cyclohexyl-hexyl]-propansulfonsäureamid, Hydrochlorid, F. 116° (Zers.)
N-[4S-Hydroxy-5S-(4-BOC-amino-piperidinocarbonyl-Phe-His-amino)-6-cyclohexyl-hexyl]-1-methyl-ethansulfonsäureamid, F. 114-116°
N-[4S-Hydroxy-5S-(4-BOC-amino-piperidinocarbonyl-Mal-His-amino)-6-cyclohexyl-hexyl]-1-methyl-ethansulfonsäureamid, F. 107-109°
N-[4S-Hydroxy-5S-(2S-benzyl-3-tert.-butylsulfonyl-propionyl-His-amino)-6-cyclohexyl-hexyl]-1-methyl-ethansulfonsäureamid, Hydrochlorid, F. 122-123°
N-[4S-Hydroxy-5S-(2R-benzyl-3-tert.-butylsulfonyl-propionyl-His-amino)-6-cyclohexyl-hexyl]-1-methyl-ethansulfonsäureamid, Hydrochlorid, F. 97-98°
N-[4S-Hydroxy-5S-(3-BOC-amino-3-methylbutyryl-Mal-His-amino)-6-cyclohexyl-hexyl]-propansulfonsäureamid, F. 132-134°
N-[4S-Hydroxy-5S-(2S-benzyl-3-tert.-butylsulfonyl-propionyl-His-amino)-6-cyclohexyl-hexyl]-N′-isopropyl-harnstoff, Hydrochlorid, F. 170-171°

N-[4S-Hydroxy-5S-(2R-benzyl-3-tert.-butylsulfonyl-propionyl-His-amino)-6-cyclohexyl-hexyl]-N'-isopropyl-harnstoff, Hydrochlorid, F. 176° (Zers.)

N-[4S-Hydroxy-5S-(4-BOC-aminopiperidinocarbonyl-Mal-His-amino)-6-cyclohexyl-hexyl]-N'-isopropyl-harnstoff, Hydrochlorid, F. 185-186°

N-[4S-Hydroxy-5S-(4-BOC-aminopiperidinocarbonyl-Phe-His-amino)-6-cyclohexyl-hexyl]-N'-isopropyl-harnstoff, Hydrochlorid, F. 186-187°

N-[2R-Methyl-4S-hydroxy-5S-(3-BOC-amino-3-methylbutyryl-Phe-His-amino)-6-cyclohexyl-hexyl]-propansulfonsäureamid

N-[2R-Methyl-4S-hydroxy-5S-(3-BOC-amino-3-methylbutyryl-Mal-His-amino)-6-cyclohexyl-hexyl]-propansulfonsäureamid

N-[2R-Methyl-4S-hydroxy-5S-(3-BOC-amino-3-methylbutyryl-Phe-N-Me-His-amino)-6-cyclohexyl-hexyl]-propansulfonsäureamid

N-[2R-Methyl-4S-hydroxy-5S-(3-BOC-amino-3-methylbutyryl-Phe-His-amino)-6-cyclohexyl-hexyl]-1-methylethansulfonsäureamid

N-[2R-Methyl-4S-hydroxy-5S-(3-BOC-amino-3-methylbutyryl-Mal-His-amino)-6-cyclohexyl-hexyl]-1-methylethansulfonsäureamid

N-[2R-Methyl-4S-hydroxy-5S-(3-BOC-amino-3-methylbutyryl-Phe-N-Me-His-amino)-6-cyclohexyl-hexyl]-1-methylethansulfonsäureamid

N-[2R-Methyl-4S-hydroxy-5S-(3-BOC-amino-3-methylbutyryl-Phe-His-amino)-6-cyclohexyl-hexyl]-cyclohexan-sulfonsäureamid

N-[2R-Methyl-4S-hydroxy-5S-(3-BOC-amino-3-methylbutyryl-Phe-Mal-amino)-6-cyclohexyl-hexyl]-cyclohexan-sulfonsäureamid

N-[2R-Methyl-4S-hydroxy-5S-(3-BOC-amino-3-methylbutyryl-Phe-His-amino)-6-cyclohexyl-hexyl]-N'-ethyl-harnstoff

N-[2R-Methyl-4S-hydroxy-5S-(3-BOC-amino-3-methylbutyryl-Phe-Mal-amino)-6-cyclohexyl-hexyl]-N'-ethyl-harnstoff

N-[2R-Methyl-4S-hydroxy-5S-(3-BOC-amino-3-methylbutyryl-Phe-N-Me-His-amino)-6-cyclohexyl-hexyl]-N'-isopropylharnstoff

N-[2R-Methyl-4S-hydroxy-5S-(4-BOC-amino-piperidinocarbonyl-Phe-His-amino)-6-cyclohexyl-hexyl]-1-methylethansulfonsäureamid

N-[2R-Methyl-4S-hydroxy-5S-(4-BOC-amino-piperidinocarbonyl-Mal-His-amino)-6-cyclohexyl-hexyl]-1-methylethansulfonsäureamid

N-[2R-Methyl-4S-hydroxy-5S-(4-BOC-amino-piperidinocarbonyl-Phe-His-amino)-6-cyclohexyl-hexyl]-cyclohexansulfonsäureamid

N-[2R-Methyl-4S-hydroxy-5S-(4-BOC-amino-piperidinocarbonyl-Mal-His-amino)-6-cyclohexyl-hexyl]-cyclohexansulfonsäureamid

N-[2R-Methyl-4S-hydroxy-5S-(4-BOC-amino-piperidinocarbonyl-Phe-His-amino)-6-cyclohexyl-hexyl]-N'-ethyl-harnstoff

N-[2R-Methyl-4S-hydroxy-5S-(4-BOC-amino-piperidinocarbonyl-Mal-His-amino)-6-cyclohexyl-hexyl]-N'-ethyl-harnstoff

N-[2R-Methyl-4S-hydroxy-5S-(4-BOC-amino-piperidinocarbonyl-Phe-His-amino)-6-cyclohexyl-hexyl]-N'-isopropylharnstoff

N-[2R-Methyl-4S-hydroxy-5S-(4-BOC-amino-piperidinocarbonyl-Mal-His-amino)-6-cyclohexyl-hexyl]-N'-isopropylharnstoff

N-[2R-Methyl-4S-hydroxy-5S-(4-BOC-amino-piperidinocarbonyl-Phe-His-amino)-6-cyclohexyl-hexyl]-N'-phenyl-harnstoff

N-[2R-Methyl-4S-hydroxy-5S-(4-BOC-amino-piperidinocarbonyl-Mal-His-amino)-6-cyclohexyl-hexyl]-N'-phenyl-harnstoff

N-[2R-Methyl-4S-hydroxy-5S-(4-BOC-amino-piperidinocarbonyl-Phe-His-amino)-6-cyclohexyl-hexyl]-3-methylbutyramid

N-[2R-Methyl-4S-hydroxy-5S-(4-BOC-amino-piperidinocarbonyl-Mal-His-amino)-6-cyclohexyl-hexyl]-3-methylbutyramid

N-[2R-Methyl-4S-hydroxy-5S-(2R-benzyl-4-(4-BOC-amino-piperidinocarbonyl)-propionyl-His-amino)-6-cyclohexyl-hexyl]-propansulfonsäureamid

N-[2R-Methyl-4S-hydroxy-5S-(2S-benzyl-4-(4-BOC-amino-piperidinocarbonyl)-propionyl-His-amino)-6-cyclohexyl-hexyl]-propansulfonsäureamid

N-[2R-Methyl-4S-hydroxy-5S-(2R-(1-naphthylmethyl)-3-(4-BOC-amino-piperidinocarbonyl)-propionyl-His-amino)-6-cyclohexyl-hexyl]-propansulfonsäureamid

N-[2R-Methyl-4S-hydroxy-5S-(2S-(1-naphthylmethyl)-3-(4-BOC-amino-piperidinocarbonyl)-propionyl-His-amino)-6-cyclohexyl-hexyl]-propansulfonsäureamid

N-[2R-Methyl-4S-hydroxy-5S-(2R-benzyl-5-N-BOC-N-methyl-amino-pentanoyl-His-amino)-6-cyclohexyl-hexyl]-propansulfonsäureamid

N-[2R-Methyl-4S-hydroxy-5S-(2S-benzyl-5-N-BOC-N-methyl-amino-pentanoyl-His-amino)-6-cyclohexyl-hexyl]-propansulfonsäureamid

N-[2R-Methyl-4S-hydroxy-5S-(2R-benzyl-5-N-BOC-N-methyl-amino-pentanoyl-His-amino)-6-cyclohexyl-hexyl]-1-methylethansulfonsäureamid

N-[2R-Methyl-4S-hydroxy-5S-(2S-benzyl-5-N-BOC-N-methyl-amino-pentanoyl-His-amino)-6-cyclohexyl-hexyl]-1-methylethansulfonsäureamid

N-[2R-Methyl-4S-hydroxy-5S-(2R-benzyl-5-N-BOC-N-methyl-amino-pentanoyl-His-amino)-6-cyclohexyl-hexyl]-N´-ethyl-harnstoff

N-[2R-Methyl-4S-hydroxy-5S-(2S-benzyl-5-N-BOC-N-methyl-amino-pentanoyl-His-amino)-6-cyclohexyl-hexyl]-N´-ethyl-harnstoff

N-[4S-Hydroxy-5S-(2R-benzyl-3-tert.-butylsulfonyl-propionyl-His-amino)-6-cyclohexyl-hexyl]-cyclohexansulfonsäureamid

N-[4S-Hydroxy-5S-(2R-benzyl-3-tert.-butylsulfonyl-propionyl-His-amino)-6-cyclohexyl-hexyl]-cyclohexansulfonsäureamid

N-[4S-Hydroxy-5S-(2R-benzyl-3-tert.-butylsulfonyl-propionyl-His-amino)-6-cyclohexyl-hexyl]-N´-isopropylharnstoff

N-[4S-Hydroxy-5S-(2S-benzyl-3-tert.-butylsulfonyl-propionyl-His-amino)-6-cyclohexyl-hexyl]-N´-isopropylharnstoff

N-[2R-Methyl-4S-hydroxy-5S(2R-benzyl-3-tert.-butylsulfonyl-propionyl-His-amino)-6-cyclohexyl-hexyl]-1-methyl-ethansulfonsäureamid

N-[2R-Methyl-4S-hydroxy-5S(2S-benzyl-3-tert.-butylsulfonyl-propionyl-His-amino)-6-cyclohexyl-hexyl]-1-methyl-ethansulfonsäureamid

N-[2R-Methyl-4S-hydroxy-5S(2R-benzyl-3-tert.-butylsulfonyl-propionyl-His-amino)-6-cyclohexyl-hexyl]-cyclohexansulfonsäureamid

N-[2R-Methyl-4S-hydroxy-5S(2S-benzyl-3-tert.-butylsulfonyl-propionyl-His-amino)-6-cyclohexyl-hexyl]-cyclohexansulfonsäureamid

N-[2R-Methyl-4S-hydroxy-5S(2R-benzyl-3-tert.-butylsulfonyl-propionyl-His-amino)-6-cyclohexyl-hexyl]-N´-ethyl-harnstoff

N-[2R-Methyl-4S-hydroxy-5S(2S-benzyl-3-tert.-butylsulfonyl-propionyl-His-amino)-6-cyclohexyl-hexyl]-N´-ethyl-harnstoff

N-[2R-Methyl-4S-hydroxy-5S(2R-benzyl-3-tert.-butylsulfonyl-propionyl-His-amino)-6-cyclohexyl-hexyl]-N´-isopropylharnstoff

N-[2R-Methyl-4S-hydroxy-5S(2S-benzyl-3-tert.-butylsulfonyl-propionyl-His-amino)-6-cyclohexyl-hexyl]-N´-isopropylharnstoff

4S-Hydroxy-5S-(2R-benzyl-3-tert.-butylsulfonyl-propionyl-His-amino)-6-cyclohexyl-hexansäurenitril

4S-Hydroxy-5S-(2S-benzyl-3-tert.-butylsulfonyl-propionyl-His-amino)-6-cyclohexyl-hexansäurenitril

2R-Methyl-4S-hydroxy-5S-(2R-benzyl-3-tert.-butylsulfonyl-propionyl-His-amino)-6-cyclohexyl-hexansäurenitril

2S-Methyl-4S-hydroxy-5S-(2R-benzyl-3-tert.-butylsulfonyl-propionyl-His-amino)-6-cyclohexyl-hexansäurenitril

2R-Methyl-4S-hydroxy-5S-(2S-benzyl-3-tert.-butylsulfonyl-propionyl-His-amino)-6-cyclohexyl-hexansäurenitril

2S-Methyl-4S-hydroxy-5S-(2S-benzyl-3-tert.-butylsulfonyl-propionyl-His-amino)-6-cyclohexyl-hexansäurenitril

N-[2R-Methyl-4S-hydroxy-5S-(2R-benzyl-heptanoyl-His-amino)-6-cyclohexyl-hexyl]-propansulfonsäureamid

N-[2R-Methyl-4S-hydroxy-5S-(2S-benzyl-heptanoyl-His-amino)-6-cyclohexyl-hexyl]-propansulfonsäureamid

N-[2R-Methyl-4S-hydroxy-5S-(2R-(1-naphthylmethyl)-3-morpholinocarbonyl-propionyl-His-amino)-6-cyclohexyl-hexyl]-propansulfonsäureamid

N-[2R-Methyl-4S-hydroxy-5S-(2S-(1-naphthylmethyl)-3-morpholinocarbonyl-propionyl-His-amino)-6-cyclohexyl-hexyl]-propansulfonsäureamid

N-[2R-Methyl-4S-hydroxy-5S-(2R-benzyl-3-tert.-butylthiopropionyl-His-amino)-6-cyclohexyl-hexyl]-propansulfonsäureamid

N-[2R-Methyl-4S-hydroxy-5S-(2S-benzyl-3-tert.-butylthiopropionyl-His-amino)-6-cyclohexyl-hexyl]-propansulfonsäureamid

N-[2R-Methyl-4S-hydroxy-5S-(2R-benzyl-3-tert.-butylsulfinyl-propionyl-His-amino)-6-cyclohexyl-hexyl]-propansulfonsäureamid

N-[2R-Methyl-4S-hydroxy-5S-(2S-benzyl-3-tert.-butylsulfinyl-propionyl-His-amino)-6-cyclohexyl-hexyl]-propansulfonsäureamid
N-[2R-Methyl-4S-hydroxy-5S-(2R-benzyl-4-oxo-5,5-dimethylhexanoyl-His-amino)-6-cyclohexyl-hexyl]-propansulfonsäureamid
N-[2R-Methyl-4S-hydroxy-5S-(2S-benzyl-4-oxo-5,5-dimethylhexanoyl-His-amino)-6-cyclohexyl-hexyl]-propansulfonsäureamid
N-[2R-Methyl-4S-hydroxy-5S-(2R-benzyl-4-hydroxy-5,5-dimethyl-hexanoyl-His-amino)-6-cyclohexyl-hexyl]-propansulfonsäureamid
N-[2R-Methyl-4S-hydroxy-5S-(2S-benzyl-4-hydroxy-5,5-dimethyl-hexanoyl-His-amino)-6-cyclohexyl-hexyl]-propansulfonsäureamid
N-[2R-Methyl-4S-hydroxy-5S-(4-dimethylamino-piperidinocarbonyl-Phe-His-amino)-6-cyclohexyl-hexyl]-propansulfonsäureamid
N-[2R-Methyl-4S-hydroxy-5S-(4-dimethylamino-piperidinocarbonyl-Mal-His-amino)-6-cyclohexyl-hexyl]-propansulfonsäureamid
N-[2R-Methyl-4S-hydroxy-5S-(N-(5-dimethylaminopentyl)-carbonyl-Phe-His-amino)-6-cyclohexyl-hexyl]-propansulfonsäureamid
N-[2R-Methyl-4S-hydroxy-5S-(N-(5-dimethylaminopentyl)-carbonyl-Phe-His-amino)-6-cyclohexyl-hexyl]-propansulfonsäureamid

Beispiel 16

Analog Beispiel 4 erhält man N-[4S-Hydroxy-5S-(4-BOC-amino-piperidinocarbonyl-Phe-βAla-amino)-6-cyclohexyl-hexyl]-propansulfonsäureamid, F. 96-97°.

Beispiel 17

Analog Beispiel 9 erhält man aus den entsprechenden BOC-Derivaten:
N-[2R-Methyl-4S-hydroxy-5S-(4-amino-piperidinocarbonyl-Phe-His-amino)-6-cyclohexyl-hexyl]-propansulfonsäureamid, Hydrochlorid, F. 173° (Zers.)
N-[2S-Methyl-4S-hydroxy-5S-(4-amino-piperidinocarbonyl-Phe-His-amino)-6-cyclohexyl-hexyl]-propansulfonsäureamid,
N-[2R-Methyl-4S-hydroxy-5S-(4-amino-piperidinocarbonyl-Mal-His-amino)-6-cyclohexyl-hexyl]-propansulfonsäureamid, Dihydrochlorid, F. 136° (Zers.)
N-[4S-Hydroxy-5S-(4-amino-piperidinocarbonyl-Phe-His-amino)-6-cyclohexyl-hexyl]-1-methyl-ethansulfonsäureamid, Dihydrochlorid, F. 145° (Zers.)
N-[4S-Hydroxy-5S-(4-amino-piperidinocarbonyl-Mal-His-amino)-6-cyclohexyl-hexyl]-1-methyl-ethansulfonsäureamid, Dihydrochlorid, F. 173° (Zers.)
N-[4S-Hydroxy-5S-(3-amino-3-methylbutyryl-Mal-His-amino)-6-cyclohexyl-hexyl]-propansulfonsäureamid, Dihydrochlorid, F. 177° (Zers.)
N-[4S-Hydroxy-5S-(4-amino-piperidinocarbonyl-Mal-His-amino)-6-cyclohexyl-hexyl]-N´-isopropyl-harnstoff, Dihydrochlorid, F. 209-210°
N-[4S-Hydroxy-5S-(4-amino-piperidinocarbonyl-Phe-His-amino)-6-cyclohexyl-hexyl]-N´-isopropyl-harnstoff, Dihydrochlorid, F. 199° (Zers.)
N-[2R-Methyl-4S-hydroxy-5S-(3-amino-3-methylbutyryl-Phe-His-amino)-6-cyclohexyl-hexyl]-propansulfonsäureamid
N-[2R-Methyl-4S-hydroxy-5S-(3-amino-3-methylbutyryl-Mal-His-amino)-6-cyclohexyl-hexyl]-propansulfonsäureamid
N-[2R-Methyl-4S-hydroxy-5S-(3-amino-3-methylbutyryl-Phe-N-Me-His-amino)-6-cyclohexyl-hexyl]-propansulfonsäureamid
N-[2R-Methyl-4S-hydroxy-5S-(3-amino-3-methylbutyryl-Phe-His-amino)-6-cyclohexyl-hexyl]-1-methyl-ethansulfonsäureamid
N-[2R-Methyl-4S-hydroxy-5S-(3-amino-3-methylbutyryl-Mal-His-amino)-6-cyclohexyl-hexyl]-1-methyl-ethansulfonsäureamid
N-[2R-Methyl-4S-hydroxy-5S-(3-amino-3-methylbutyryl-Phe-N-Me-His-amino)-6-cyclohexyl-hexyl]-1-methyl-ethansulfonsäureamid
N-[2R-Methyl-4S-hydroxy-5S-(3-amino-3-methylbutyryl-Phe-His-amino)-6-cyclohexyl-hexyl]-cyclohexansulfonsäureamid

N-[2R-Methyl-4S-hydroxy-5S-(3-amino-3-methylbutyryl-Phe-Mal-amino)-6-cyclohexyl-hexyl]-cyclohexansulfonsäureamid

N-[2R-Methyl-4S-hydroxy-5S-(3-amino-3-methylbutyryl-Phe-His-amino)-6-cyclohexyl-hexyl]-N'-ethyl-harnstoff

N-[2R-Methyl-4S-hydroxy-5S-(3-amino-3-methylbutyryl-Phe-Mal-amino)-6-cyclohexyl-hexyl]-N'-ethyl-harnstoff

N-[2R-Methyl-4S-hydroxy-5S-(3-amino-3-methylbutyryl-Phe-N-Me-His-amino)-6-cyclohexyl-hexyl]-N'-isopropylharnstoff

N-[2R-Methyl-4S-hydroxy-5S-(4-amino-piperidinocarbonyl-Phe-His-amino)-6-cyclohexyl-hexyl]-1-methylethansulfonsäureamid

N-[2R-Methyl-4S-hydroxy-5S-(4-amino-piperidinocarbonyl-Mal-His-amino)-6-cyclohexyl-hexyl]-1-methylethansulfonsäureamid

N-[2R-Methyl-4S-hydroxy-5S-(4-amino-piperidinocarbonyl-Phe-His-amino)-6-cyclohexyl-hexyl]-cyclohexansulfonsäureamid

N-[2R-Methyl-4S-hydroxy-5S-(4-amino-piperidinocarbonyl-Mal-His-amino)-6-cyclohexyl-hexyl]-cyclohexansulfonsäureamid

N-[2R-Methyl-4S-hydroxy-5S-(4-amino-piperidinocarbonyl-Phe-His-amino)-6-cyclohexyl-hexyl]-N'-ethyl-harnstoff

N-[2R-Methyl-4S-hydroxy-5S-(4-amino-piperidinocarbonyl-Mal-His-amino)-6-cyclohexyl-hexyl]-N'-ethyl-harnstoff

N-[2R-Methyl-4S-hydroxy-5S-(4-amino-piperidinocarbonyl-Phe-His-amino)-6-cyclohexyl-hexyl]-N'-isopropyl-harnstoff

N-[2R-Methyl-4S-hydroxy-5S-(4-amino-piperidinocarbonyl-Mal-His-amino)-6-cyclohexyl-hexyl]-N'-isopropyl-harnstoff

N-[2R-Methyl-4S-hydroxy-5S-(4-amino-piperidinocarbonyl-Phe-His-amino)-6-cyclohexyl-hexyl]-N'-phenyl-harnstoff

N-[2R-Methyl-4S-hydroxy-5S-(4-amino-piperidinocarbonyl-Mal-His-amino)-6-cyclohexyl-hexyl]-N'-phenyl-harnstoff

N-[2R-Methyl-4S-hydroxy-5S-(4-amino-piperidinocarbonyl-Phe-His-amino)-6-cyclohexyl-hexyl]-3-methylbutyramid

N-[2R-Methyl-4S-hydroxy-5S-(4-amino-piperidinocarbonyl-Mal-His-amino)-6-cyclohexyl-hexyl]-3-methylbutyramid

N-[2R-Methyl-4S-hydroxy-5S-(2R-benzyl-3-(4-amino-piperidinocarbonyl)-propionyl-His-amino)-6-cyclohexyl-hexyl]-propansulfonsäureamid

N-[2R-Methyl-4S-hydroxy-5S-(2S-benzyl-3-(4-amino-piperidinocarbonyl)-propionyl-His-amino)-6-cyclohexyl-hexyl]-propansulfonsäureamid

N-[2R-Methyl-4S-hydroxy-5S-(2R-(1-naphthylmethyl)-3-(4-amino-piperidinocarbonyl)-propionyl-His-amino)-6-cyclohexyl-hexyl]-propansulfonsäureamid

N-[2R-Methyl-4S-hydroxy-5S-(2S-(1-naphthylmethyl)-3-(4-amino-piperidinocarbonyl)-propionyl-His-amino)-6-cyclohexyl-hexyl]-propansulfonsäureamid

N-[2R-Methyl-4S-hydroxy-5S-(2R-benzyl-5-N-methyl-amino-pentanoyl-His-amino)-6-cyclohexyl-hexyl]-propansulfonsäureamid

N-[2R-Methyl-4S-hydroxy-5S-(2S-benzyl-5-N-methyl-amino-pentanoyl-His-amino)-6-cyclohexyl-hexyl]-propansulfonsäureamid

N-[2R-Methyl-4S-hydroxy-5S-(2R-benzyl-5-N-methyl-amino-pentanoyl-His-amino)-6-cyclohexyl-hexyl]-1-methylethansulfonsäureamid

N-[2R-Methyl-4S-hydroxy-5S-(2S-benzyl-5-N-methyl-amino-pentanoyl-His-amino)-6-cyclohexyl-hexyl]-1-methylethansulfonsäureamid

N-[2R-Methyl-4S-hydroxy-5S-(2R-benzyl-5-N-methyl-amino-pentanoyl-His-amino)-6-cyclohexyl-hexyl]-N'-ethyl-harnstoff

N-[2R-Methyl-4S-hydroxy-5S-(2S-benzyl-5-N-methyl-amino-pentanoyl-His-amino)-6-cyclohexyl-hexyl]-N'-ethyl-harnstoff

Beispiel 18

Ein Gemisch von 730 mg N-[2R-Methyl-4S-hydroxy-5S-(4-amino-piperidino-carbonyl-Phe-His-amino)-6-cyclohexyl-hexyl]-propansulfonsäureamid, 90 mg S-Methylisothioharnstoff, 5 ml Ethanol und 5 ml Wasser wird 2 Std. bei 50° gerührt. Nach üblicher Aufarbeitung erhält man N-[2R-Methyl-4S-hydroxy-5S-(4-guanidino-piperidinocarbonyl-Phe-His-amino)-6-cyclohexyl-hexyl]-propansulfonsäureamid.

Analog erhält man N-[2R-Methyl]-4S-hydroxy-5S-(4-guanidino-piperidinocarbonyl-Mal-His-amino)-6-cyclohexyl-hexyl]-propansulfonsäureamid.

Beispiel 19

Ein Gemisch von 730 mg N-[2R-Methyl-4S-hydroxy-5S-(4-amino-piperidino-carbonyl-Phe-His-amino)-6-cyclohexyl-hexyl]-propansulfonsäureamid, 115 mg Trimethylsilylisocyanat und 10 ml THF wird 16 Std. bei 20° gerührt. Danach rührt man in Wasser ein, arbeitet wie üblich auf und erhält N-[2R-Methyl-4S-hydroxy-5S-(4-ureido-piperidinocarbonyl-Phe-His-amino)-6-cyclohexyl-hexyl]-propansulfonsäureamid.

Analog erhält man N-[2R-Methyl-4S-hydroxy-5S-(4-ureido-piperidinocarbonyl-Mal-His-amino)-6-cyclohexyl-hexyl]-propansulfonsäureamid,

sowie mit Ethylisocyanat:

N-[2R-Methyl-4S-hydroxy-5S-(4-N'-ethylureido-piperidino-carbonyl-Phe-His-amino)-6-cyclohexyl-hexyl]-propansulfonsäureamid

N-[2R-Methyl-4S-hdyroxy-5S-(4-N'-ethylureido-piperidino-carbonyl-Mal-His-amino)-6-cyclohexyl-hexyl]-propansulfonsäureamid.

Die nachfolgenden Beispiele betreffen pharmazeutische Zubereitungen.

Beispiel A: Tabletten

Ein Gemisch von 1 kg 2S-Isopropyl-4S-hydroxy-5S-(2-benzyl-4-oxo-5,5-dimethyl-hexanoyl-His-amino)-6-cyclohexyl-hexyl-hexansäurenitril, 4 kg Lactose, 1,2 kg Maisstärke, 200 g Talk und 100 g Magnesiumstearat wird in üblicher Weise zu Tabletten verpreßt, derart daß jede Tablette 100 mg Wirkstoff enthält.

Beispiel B: Dragees

Analog Beispiel A werden Tabletten gepreßt, die anschließend in üblicher Weise mit einem Überzug aus Saccharose, Maisstärke, Talk, Tragant und Farbstoff überzogen werden.

Beispiel C: Kapseln

500 g N-[2S-Isopropyl-4S-hydroxy-5S-(2-benzyl-3-tert.-butylsulfonyl-propionyl-His-amino)-6-cyclohexyl-hexyl]-butansulfonsäureamid-hydrochlorid (F. 109°) werden in üblicher Weise in Hartgelatinekapseln gefüllt, so daß jede Kapsel 500 mg Wirkstoff enthält.

Beispiel D: Injektionsgläser

Eine Lösung von 100 g N-[2S-Isopropyl-4S-hydroxy-5S-(4-aminopiperidinocarbonyl-Phe-His-amino)-6-cyclohexyl-hexyl]-propansulfonsäureamid-dihydrochlorid in 4 l zweifach destilliertem Wasser wird mit 2n Salzsäure auf pH 6,5 eingestellt, steril filtriert und in Injektionsgläser abgefüllt. Man lyophilisiert unter sterilen Bedingungen und verschließt steril. Jedes Injektionsglas enthält 50 mg Wirkstoff.

Beispiel E: Suppositorien

Man schmilzt ein Gemisch von 50 g N-[2S-Isopropyl-4S-hydroxy-5S-(2-benzyl-3-tert.-butylsulfonyl-propionyl-His-amino)-6-cyclohexyl-hexyl]-methansulfonsäureamid-hydrochlorid (F. 112°) mit 10 g Sojalecithin und 140 g Kakaobutter, gießt in Formen und läßt erkalten. Jedes Suppositorium enthält 250 mg Wirkstoff.

**Ansprüche**

1. Aminosäurederivate der Formel I

R'-Z-NR²-CHR³-CR⁴-CH₂-CR⁵R⁶-Y    I

worin

R¹ H, R⁷-O-C$_m$H$_{2m}$-CO-, R⁷-C$_m$H$_{2m}$-O-CO-, R⁷-C$_m$H$_{2m}$-CO-, R⁷-SO₂-, R⁸R⁹N-C$_m$H$_{2m}$-CO-, R¹⁰-NH-C(=NH)-NH-C$_m$H$_{2m}$-CO-, R⁸OOC-C$_m$H$_{2m}$-CO-, R⁸O₃S-C$_m$H$_{2m}$-CO- oder R¹¹-C$_m$H$_{2m}$-(T)$_s$-(V)$_y$-C$_n$H$_{2n}$-L(R⁷-C $_p$H$_{2p}$)-C$_r$H$_{2r}$-CO-,

Z 1 bis 4 peptidartig miteinander verbundene Aminosäurereste ausgewählt aus der Gruppe bestehend aus Abu, Ada, Ala, βAla, Arg, Asn, Asp, Bia, Cal, Dab, Gln, Glu, Gly, His, N(im)-A-His, Hph, Ile, Leu, tert.-Leu Lys, Mal, Met, αNal, βNal, Nbg, Nle, Orn, Phe, Pia, Pro, Pyr, Ser, Thr, Tia, Tic, Trp, Tyr und Val,

Y -CN, -CH₂-NR¹²R¹³, -CH₂-NR¹²-SO₂R¹⁴, -CH₂-NR¹²-COR¹⁴, -CH₂-NR¹²-CO-NH-R¹⁴, oder -CH₂-NR¹²-CS-NH-R¹⁴,

R², R⁵, R⁶, R⁸, R⁹ und R¹³ jeweils H oder A,

R³, R⁷, R¹¹, R¹² und R¹⁴ jeweils H, A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

R¹¹ auch R⁸O-, R⁸R⁹N-, R⁸OOC- oder A₃N$^⊕$ An$^⊖$,

R⁴ (H, OH), (H, NH₂) oder =O,

R¹⁰ H, A oder CN,

L CH oder N,

T O, NH oder NA,

V CHOH, CO, S, SO oder SO₂,

R⁸R⁹N und NR¹²R¹³ auch jeweils eine unsubstituierte oder eine durch A, OH, NH₂, NHA, NA₂, NHAc, NH-CO-C$_x$H$_{2x}$-O-R¹⁵, NH-CO-O-C$_x$H$_{2x}$-R¹⁵, Hydroxyalkyl, COOH, COOA, CONH₂, Aminoalkyl, HAN-alkyl, A₂N-alkyl, A₃N$^⊕$alkyl An$^⊖$, NH-CO-NH₂, NH-CO-NHA, Guanidinyl oder Guanidinyl-alkyl substituierte Pyrrolidino-, Piperidino-, Morpholino- oder Piperazinogruppe,

R¹⁵ A oder Ar-alkyl,

s und y jeweils 0 oder 1,

m, n, p, r und x jeweils 0, 1, 2, 3, 4, 5, 6, 7, 8, 9 oder 10,

Ar unsubstituiertes oder ein- oder mehrfach durch A, OA, Hal, CF₃, OH, NO₂, Hydroxyalkyl, NH₂, NHA, NA₂, NHAc, SA, SO-A, SO₂-A, SO₂NH₂, SO₂NHA, COOH, COOA, CONH₂, CN, Aminoalkyl, HAN-alkyl, A₂N-alkyl, A₃N$^⊕$ alkyl An$^⊖$ und/oder Guanidinyl-alkyl substituiertes Phenyl oder unsubstituiertes Naphthyl,

Het einen gesättigten oder ungesättigten 5-oder 6-gliedrigen heterocyclischen Rest mit 1-4 N-, O- und/oder S-Atomen, der mit einem Benzolring kondensiert und/oder ein-oder mehrfach durch A, OA, Hal, CF₃, OH, No₂, Carbonylsauerstoff, NH₂, NHA, NA₂, NHAc, SA, SO-A, SO₂-A, SO₂NH₂, SO₂NHA, COOH, COOA, CONH₂, CN, NH-SO₂-A, Ar, Ar-alkyl, Ar-alkenyl, Hydroxyalkyl, Amino-alkyl, HAN-alkyl und/oder A₂N-alkyl substituiert sein kann und/oder dessen N-und/oder S-Heteroatome auch oxydiert sein können,

Hal F, Cl, Br oder J,

Ac A-CO-, Ar-CO-, Ar-alkyl-CO- oder A-NH-CO-,

An$^⊕$ ein Anion, das auch fehlen kann, wenn stattdessen eine in der Verbindung der Formel I enthaltene Carboxygruppe in Form eines Carboxylatanions vorliegt,

-alkyl- eine Alkylengruppe mit 1-8 C-Atomen und

A Alkyl mit 1-8 C-Atomen bedeuten,

worin ferner an Stelle einer oder mehrerer -NH-CO-Gruppen auch eine oder mehrere -NA-CO-Gruppen stehen können,

sowie deren Salze.

2.

   a) N-[2-Isopropyl-4-hydroxy-5-(4-aminopiperidinocarbonyl-Phe-His-amino)-6-cyclohexyl-hexyl]-propansulfonsäureamid;

   b) N-[2-Isopropyl-4-hydroxy-5-(2-benzyl-3-tert.-butylsulfonyl-propionyl-His-amino)-6-cyclohexyl-hexyl]-methansulfonsäureamid.

3. Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Carbonsäure der Formel II

R'-G'-OH    II

worin G$^1$

(a) fehlt,

(b) Z,

(c) Z$^1$ bedeutet

oder eines ihrer reaktionsfähigen Derivate

mit einer Aminoverbindung der Formel III

H-G$^2$-NR$^2$-CHR$^3$-CR$^4$-CH$_2$-CR$^5$R$^6$-Y    III

worin G$^2$

(a) Z bedeutet,

(b) fehlt,

(c) Z$^2$ bedeutet und

Z$^1$ + Z$^2$ zusammen Z bedeuten,

umsetzt,

und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine freie Aminogruppe durch Behandeln mit einem acylierenden Mittel acyliert und/oder zur Herstellung einer Verbindung der Formel I, R$^4$ = (H, OH) oder (H, NH$_2$), ein Aminoketosäure-derivat der Formel I, R$^4$ = O, reduziert oder reduktiv aminiert und/oder einen Rest R$^1$ in einen anderen Rest R$^1$ umwandelt und/oder eine Verbindung der Formel I durch Behandeln mit einer Säure in eines ihrer Salze überführt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I oder von deren physiologisch unbedenklichen Salzen zur Herstellung eines Arzneimittels.

7. Verwendung von Verbindungen der Formel I oder von deren physiologisch unbedenklichen Salzen bei der Bekämpfung der reninabhängigen Hypertension oder des Hyperaldosteronismus.